# FASCICULE DE BREVET EUROPEEN

(11) **EP 2 464 747 B1**
(45) Date de publication et mention de la délivrance du brevet: **10.04.2019**
(21) Numéro de dépôt: 10773710.8
(22) Date de dépôt: 13.08.2010
(51) Int. Cl.: C12Q 1/6827, C12Q 1/6853

(54) **PROCEDE DE DETECTION D'UN ADN CIRCULARISE ET UTILISATION DE CE PROCEDE POUR LA DETECTION DE MUTATIONS**
VERFAHREN ZUR DETEKTION VON ZIRKULARISIERTER DNA UND DEREN VERWENDUNG IN METHODEN ZUR DETEKTION VON MUTATIONEN
METHOD FOR DETECTION OF CIRCULARIZED DNA AND USE OF THE METHOD FOR DETECTION OF MUTATIONS

(30) Priorité: 13.08.2009 FR 0903950
(43) Date de publication de la demande: 20.06.2012
(73) Titulaire: Bockelmann, Ulrich, 75020 Paris (FR)
(72) Inventeur: BOCKELMANN, Ulrich, F-75020 Paris (FR); VIASNOFF, Virgile, F-92170 Vanves (FR); CISSE, Ismaïl, F-93300 Aubervilliers (FR)
(74) Mandataire: D Young & Co LLP
(86) Numéro de dépôt international: PCT/IB2010/053665
(87) Numéro de publication internationale: WO 2011/018774

(56) Documents cités:
- EP-A2- 0 416 817
- WO-A1-99/27137
- WO-A1-2007/100243
- WO-A2-02/24944
- WO-A2-2004/081225
- WO-A2-2006/071770
- US-A- 5 525 494
- ZHANG DAVID ET AL: "Amplification of circularizable probes for the detection of target nucleic acids and proteins.", CLINICA CHIMICA ACTA; INTERNATIONAL JOURNAL OF CLINICAL CHEMISTRY JAN 2006, vol. 363, no. 1-2, janvier 2006 (2006-01), pages 61-70, XP002575757, ISSN: 0009-8981
- BANÉR J ET AL: "More keys to padlock probes: mechanisms for high-throughput nucleic acid analysis.", CURRENT OPINION IN BIOTECHNOLOGY FEB 2001, vol. 12, no. 1, février 2001 (2001-02), pages 11-15, XP002575758, ISSN: 0958-1669
- HAUSCH F ET AL: "Multifunctional DNA conjugates for the in vitro selection of new catalysts.", NUCLEIC ACIDS RESEARCH, vol. 28, no. 8, E35, 15 avril 2000 (2000-04-15), pages I-III, XP002575759, ISSN: 1362-4962
- LIZARDI P M ET AL: "MUTATION DETECTION AND SINGLE-MOLECULE COUNTING USING ISOTHERMAL ROLLING-CIRCLE AMPLIFICATION", NATURE GENETICS, NATURE PUBLISHING GROUP, NEW YORK, US, vol. 19, no. 3, 1 July 1998 (1998-07-01), pages 225-232, XP000856939, ISSN: 1061-4036, DOI: 10.1038/898
- LI X ET AL: "Genotyping of multiple single nucleotide polymorphisms with hyperbranched rolling circle amplification and microarray", CLINICA CHIMICA ACTA, ELSEVIER BV, AMSTERDAM, NL, vol. 399, no. 1-2, 1 January 2009 (2009-01-01), pages 40-44, XP025745839, ISSN: 0009-8981, DOI: 10.1016/J.CCA.2008.08.012 [retrieved on 2008-08-23]

### SEQUENCE LISTING

<110> CENTRE NATIONAL DE LA RECHERCHE SCIENTIFIQUE UNIVERSITE PIERRE ET MARIE CURIE BOCKELMANN, Ulrich VIASNOFF, Virgile CISSE, Ismaïl
<120> PROCEDE DE DETECTION D'UN ADN CIRCULARISE ET UTILISATION DE CE PROCEDE POUR LA DETECTION DE MUTATIONS
<130> F644-200-PCT
<150> FR 09 03950
   <151> 2009-08-13
<160> 10
<170> PatentIn version 3.3
<210> 1
   <211> 40
   <212> DNA
   <213> Artificial
<220>
   <223> ADN cible de type sauvage
<400> 1
   gctactcgct gaaattaata cgactcacta ggtgccacgg 40
<210> 2
   <211> 40
   <212> DNA
   <213> Artificial
<220>
   <223> ADN cible de type mutant
<400> 2
   gctactcgct gaaattaata cgaatcacta ggtgccacgg 40
<210> 3
   <211> 82
   <212> DNA
   <213> Artificial
<220>
   <223> Sonde nucléotidique circularisable de type sauvage
<220>
   <221> primer_bind
   <222> (25)..(42)
   <223> site d'hybridation de l'amorce WTFP
<400> 3
   tcgtattaat ttcagcgagt gggatcggcg cacctgccgg aaaggccgaa ttcaacggtt 60
   gtggtctccc taacctagtg ag 82
<210> 4
   <211> 89
   <212> DNA
   <213> Artificial
<220>
   <223> Sonde nucléotidique circularisable de type mutant
<220>
   <221> primer_bind
   <222> (27)..(50)
   <223> site d'hybridation de l'amorce MutFP
<400> 4
   tcgtattaat ttcagcgagt ttctgactcg tcatgtctca gctctagtac gctgatctta 60
   gtgtcaggat acggtgtaga cctagtgat 89
<210> 5
   <211> 39
   <212> DNA
   <213> Artificial
<220>
   <223> Amorce sens de type sauvage
<220>
   <221> misc_feature
   <222> (21)..(21)
   <223> espaceur ou rien
<400> 5
   aacgtcagcc ctgccgcctg nttccggcag gtgcgccga 39
<210> 6
   <211> 42
   <212> DNA
   <213> Artificial
<220>
   <223> Amorce anti-sens de type sauvage
<400> 6
   ctaagctgta gccggagtga aggccgaatt caacggttgt gg 42
<210> 7
   <211> 47
   <212> DNA
   <213> Artificial
<220>
   <223> Amorce sens de type mutant
<220>
   <221> misc_feature
   <222> (21)..(21)
   <223> espaceur ou rien
<400> 7
   ggcggagagt cagttcgcgg ngtactagag ctgagacatg acgagtc 47
<210> 8
   <211> 44
   <212> DNA
   <213> Artificial
<220>
   <223> Amorce anti-sens de type mutant
<400> 8
   ctaagctgta gccggagtga gctgatctta gtgtcaggat acgg 44
<210> 9
   <211> 20
   <212> DNA
   <213> Artificial
<220>
   <223> sonde
<400> 9
   ccgcgaactg actctccgcc 20
<210> 10
   <211> 20
   <212> DNA
   <213> Artificial
<220>
   <223> sonde
<400> 10
   caggcggcag ggctgacgtt 20

## Description

La présente description a pour objet un procédé de détection d'un acide désoxyribonucléique (ADN) monocaténaire circularisé. La description a aussi pour objet l'utilisation de ce procédé pour la détection de mutations, par exemple d'un polymorphisme génétique d'une seule ou de plusieurs paire(s) de base.

Un ADN monocaténaire circularisé (par exemple une sonde) peut être détecté en utilisant différentes méthodes. Certaines de ces méthodes mettent en oeuvre la technique d'amplification ramifiée isotherme par cercle roulant (« hyperbranched rolling circle amplification » ou HRCA, appelée aussi « ramification amplification » ou RAM) (Lizardi et al., 1998, Nature Genetics, 19:225-232).

La HRCA est une technique qui permet la synthèse de multimères d'ADN bicaténaire (double brin) ramifiés à partir d'un ADN monocaténaire (simple brin) circularisé (substrat) dont la séquence correspond au motif élémentaire (période ou monomère) que l'on souhaite répéter. Cette réaction d'amplification peut s'effectuer à une température comprise entre 37°C et 70°C, mais s'effectue généralement à une température comprise entre 60°C et 65°C, en présence d'une ADN polymérase dépourvue d'activité exonucléasique et possédant la propriété de déplacement du brin. Cette ADN polymérase, non seulement est capable, lors de la polymérisation du brin complémentaire à un ADN matrice (c'est-à-dire l'élongation d'une amorce hybridée à un ADN matrice), de déplacer un brin d'ADN hybridé à l'ADN matrice qu'elle rencontre afin de poursuivre la polymérisation du brin complémentaire à l'ADN matrice, mais aussi est dépourvue d'une activité exonucléasique. A titre d'exemples d'une telle ADN polymérase, on peut citer l'ADN polymérase *Bst* Grand Fragment (qui correspond à un fragment de l'ADN polymérase de *Bacillus stearothermophilus*) et l'ADN polymérase *Vent* exo- (ADN polymérase exo- de *Thermococcus litoralis*). Ce processus d'amplification permet de générer 10⁹ copies ou plus de l'ADN monocaténaire circularisé en 90 minutes.

L'amplification ramifiée isotherme par cercle roulant d'un ADN monocaténaire circularisé (qui peut être obtenu par dénaturation d'un ADN bicaténaire circularisé par exemple) comprend les étapes suivantes :
a) hybridation d'une première amorce (sens) à l'ADN monocaténaire circularisé (dénommé brin négatif), et polymérisation (synthèse) d'un brin monocaténaire périodique (dénommé brin positif), dont la période correspond à la séquence de l'ADN monocaténaire circularisé, grâce à la propriété de déplacement du brin de l'ADN polymérase ; l'amorce sens comprenant ou étant constituée d'une séquence nucléotidique complémentaire à un fragment de l'ADN monocaténaire circularisé ;
b) hybridation d'une seconde amorce (antisens) à chaque période du brin monocaténaire périodique (brin positif) obtenu à l'étape a) et polymérisation de plusieurs brins périodiques complémentaires (brins négatifs) au brin positif grâce à ladite ADN polymérase qui déplace les brins négatifs hybridés (branchés) au brin positif ; l'amorce anti-sens comprenant ou étant constituée d'une séquence nucléotidique identique à un fragment de l'ADN monocaténaire circularisé ;
c) hybridation de la première amorce à chaque période des brins négatifs obtenus à l'étape b) et polymérisation de plusieurs brins périodiques complémentaires (brins positifs) au brin négatif grâce à ladite ADN polymérase qui déplace les brins positifs hybridés (branchés) au brin négatif ;
d) hybridation des amorces sens et anti-sens respectivement aux brins négatifs et positifs générés par polymérisation et déplacement des brins ;
e) obtention d'ADN bicaténaires périodiques libres ou branchés.

Il existe actuellement de nombreuses méthodes pour la détection d'un polymorphisme (mutation) génétique d'une seule paire de bases (SNP) ou de plusieurs paires de bases (voir pour revue Kim and Misra, 2007, Annu Rev Biomed Eng., 9:289-320). Pour obtenir une excellente détection d'un polymorphisme génétique d'une seule ou de plusieurs paire(s) de bases, il convient de prendre en compte un certain nombre de critères :
- une très bonne spécificité et fiabilité du mécanisme de reconnaissance des mutations,
- une bonne sensibilité en l'absence d'amplification de l'ADN génomique préalable à la détection,
- un degré élevé de multiplexage, c'est-à-dire la possibilité de cribler plusieurs milliers de mutations et d'échantillons en parallèle,
- un faible coût par détection et par patient.

La technique HRCA peut être utilisée pour la détection d'un polymorphisme génétique (Lizardi *et al.,* cité ci-dessus ; Bogard et al., 2005, Techniques de détection des mutations connues, dans Principes de biologie moléculaire en biologie clinique ; Paris : Elsevier, 720 p). En pratique, l'ADN cible sous forme monocaténaire (par exemple un ADN génomique bicaténaire dénaturé) est mis en contact avec une ou plusieurs sonde(s) (ADN monocaténaire circularisable). Si une sonde est spécifique de l'ADN cible (de type sauvage ou muté) alors elle s'hybride à sa cible. Après cette étape d'hybridation, une étape de ligation de la sonde hybridée est effectuée à l'aide, généralement, d'une ADN ligase thermostable (seule la sonde spécifiquement hybridée à sa cible étant liguée), ce qui provoque sa circularisation ; laquelle sonde est ensuite détectée en mettant en oeuvre la technique HRCA. Si un sujet est hétérozygote pour une mutation, alors les sondes hybridées et circularisées respectivement à l'allèle de type sauvage et à l'allèle muté pourront aussi être détectées simultanément.

Plusieurs variantes de cette technique ont été proposées pour améliorer la sensibilité et la spécificité de détection. A titre d'exemple, on peut citer l'utilisation d'une amorce sens ou d'une amorce anti-sens qui présente en position 5' une structure en épingle à cheveux (« *hairpin* ») contenant un fluorophore et un désactivateur de fluorescence (« *quencher* ») non fluorescent (Furaqui et al., 2001, BMC Genomics, 2:4 et Pickering et al., 2002, Nucleic Acids Research, 30:e60), l'utilisation d'une « fermeture-Eclair » moléculaire (« *molecular zipper* »), qui sert d'amorce anti-sens telle que définie ci-dessus, et qui se présente sous la forme d'une molécule d'ADN double brin dont l'un des brins est conjugué à la fluorescéine et l'autre à un désactivateur de fluorescence (Yi et al., 2006, Nucleic Acids Research, 34, e81), ou encore l'insertion d'un marqueur génétique (ou séquence code barre ; « *tag* » ou « *barcode* ») et d'un site de clivage dans la séquence nucléotidique de la sonde (cette technique étant appelée *molecular inversion probes,* MIP) (Hardenbol et al., 2003, Nature Biotechnology, 21:673-8 et Demande Internationale WO 02/057491).

Cependant, ces techniques ne sont pas entièrement satisfaisantes. En effet, la détection d'un polymorphisme génétique en utilisant ces techniques ne répond pas à tous les critères énumérés ci-dessus. Il en résulte donc un besoin d'amélioration de la technique HRCA dans ce domaine.

Le brevet WO 2006/071770 divulgue des dispositifs et des procédés automatisés permettant l'extraction de l'acide nucléique de cellules, l'amplification de segments d'acides nucléiques et la détection d'acides nucléiques. Li et al. (2009, Clinica Chimica Acta 399:40-44) divulguent l'utilisation de la technique HRCA et d'une puce à ADN 3D pour identifier différents loci comportant des SNP et pour amplifier les fragments d'échantillons génomiques.

Dans le cadre de leurs travaux, les Inventeurs ont cherché à améliorer la détection d'un polymorphisme génétique par la technique HRCA. Ils ont alors modifié la technique HRCA décrite par Lizardi *et al.* (cité ci-dessus) en ce que l'amorce sens est constituée, de son extrémité 5' vers son extrémité 3', d'une séquence nucléotidique code barre, d'un espaceur linéaire à base de polyéthylène glycol (PEG) qui permet de bloquer la polymérisation par l'ADN polymérase et d'une séquence nucléotidique apte à s'hybrider à un fragment d'un brin négatif tel que décrit ci-dessus, et en ce que l'amorce anti-sens est constituée, de son extrémité 5' vers son extrémité 3', d'un groupement terminal de type fluorophore, d'une séquence nucléotidique code barre (appelée aussi séquence de liaison si cette séquence n'est pas détectée) et d'une séquence nucléotidique apte à s'hybrider à un fragment d'un brin positif tel que décrit ci-dessus.

La mise en oeuvre d'une HRCA en utilisant ces amorces permet d'obtenir des ADN bicaténaires périodiques, sauf pour la séquence code barre qui reste monocaténaire. Ces molécules d'ADN bicaténaires périodiques peuvent alors être détectées, sans être dénaturées, par hybridation sur un support solide, par exemple une puce à ADN (*microarray*), portant comme sondes des séquences nucléotidiques complémentaires de la séquence code barre. Le fait que seule la séquence code barre soit monocaténaire (simple brin) réduit les risques d'interactions non spécifiques et d'hybridations croisées (*cross hybridation*) sur le support solide. Les Inventeurs ont dénommé cette méthode d'amplification: « *tagged hyperbranched rolling circle amplification* » (THRCA). Mis à part le couple d'amorces utilisé, les conditions réactionnelles (pH, tampon, température) de la THRCA sont similaires à celles de la HRCA décrites par Lizardi *et al.* (cité ci-dessus).

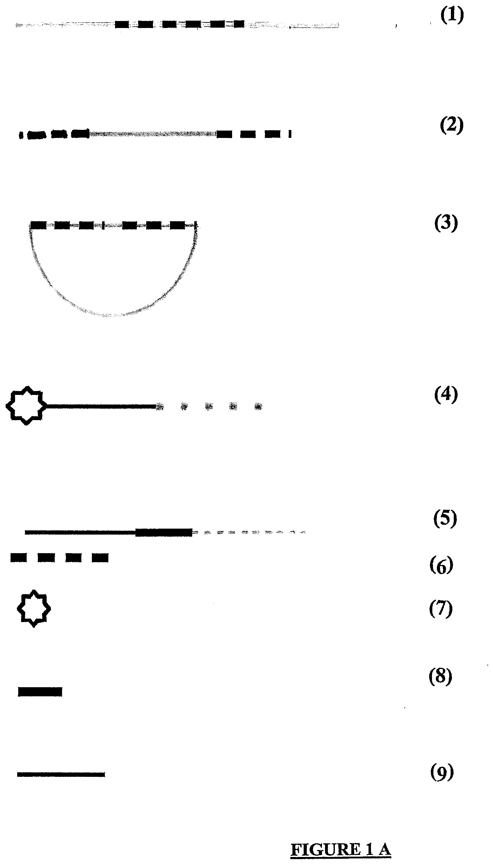

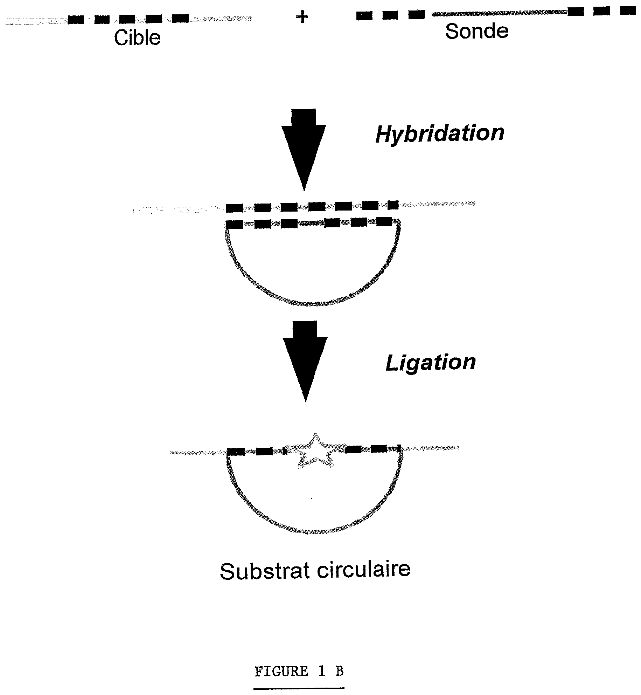

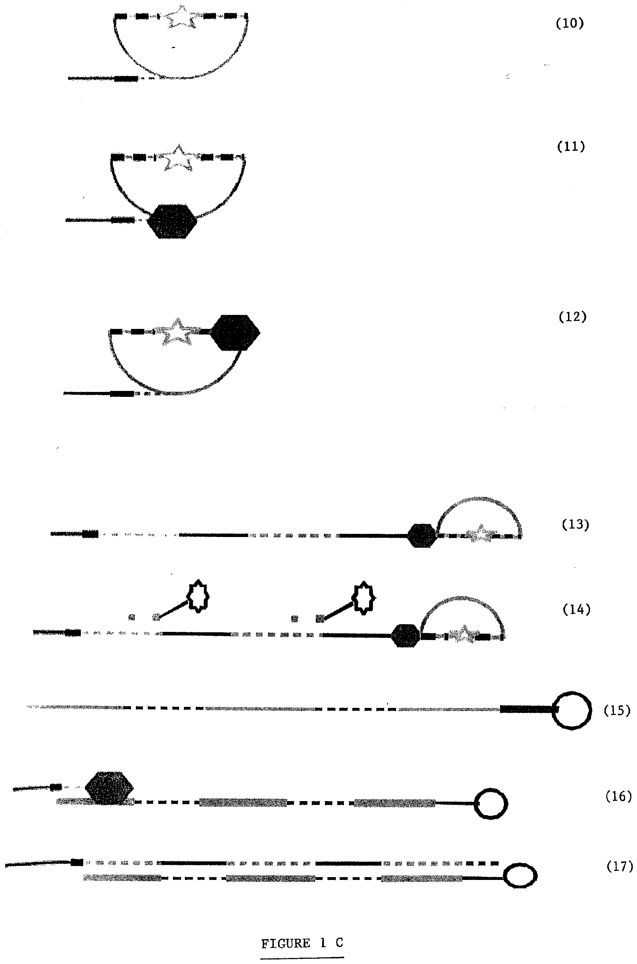

Pour faciliter la compréhension de l'invention, l'amplification par THRCA d'une sonde circularisée et hybridée à un acide nucléique cible décrite ci-avant est représentée à la Figure 1.

La détection de l'hybridation de la séquence code barre audit support solide peut ensuite s'effectuer de différentes manières ; par exemple :
- détection par fluorescence soit du fluorophore situé à l'extrémité opposée de la séquence code barre sur les ADN bicaténaires périodiques, soit de dNTPs marqués et incorporés dans les brins d'ADN polymérisés ;
- détection électronique de l'hybridation des séquences codes barres à un support (par exemple, la surface active d'un capteur électronique), comme décrite par exemple dans la Demande Internationale WO 2004/057027 ; les nombreuses molécules obtenues par amplification THRCA permettent une bonne détection de l'hybridation dans la mesure où le signal électrique augmente avec le nombre de nucléotides que contient l'ADN hybridé, par l'intermédiaire de la séquence code barre, à la surface active d'un capteur électronique ; ou
- toute autre technique de détection par hybridation dans laquelle le signal de détection de l'hybridation entre la séquence code barre et une séquence nucléotidique (sonde) complémentaire à ladite séquence code barre augmente avec le nombre de nucléotides de la molécule d'acide nucléique comprenant à son extrémité ladite séquence code barre.

Selon une variante de la THRCA, les amorces ne comprennent pas d'espaceur. Dans ce cas, avant l'étape de détection des séquences codes barres, les ADN périodiques bicaténaires peuvent être incubés avec une exonucléase ou une enzyme de restriction de manière à obtenir des ADN périodiques bicaténaires possédant une séquence code barre monocaténaire.

La présente description a en conséquence pour objet un procédé de détection d'un ADN monocaténaire circularisé par amplification ramifiée isotherme par cercle roulant (HRCA) dudit ADN monocaténaire circularisé, en présence d'une amorce sens apte à s'hybrider audit ADN monocaténaire circularisé et aux brins négatifs générés lors de la HRCA, et d'une amorce anti-sens apte à s'hybrider aux brins positifs générés lors de la HRCA, ladite HRCA générant des ADN bicaténaires périodiques, lequel procédé est caractérisé en ce que :
- ladite amorce sens est constituée de ou comprend, de son extrémité 5' vers son extrémité 3', la séquence suivante : 5'-(F1)ₙ₁-T1-(E1)ₘ₁-A1-3', dans laquelle :
   ∘ F1 représente un groupement terminal, choisi parmi un marqueur et un agent de couplage,
   ∘ T1 représente une séquence nucléotidique code barre, de préférence constituée de 6 à 30 nucléotides, de préférence de 10 à 25 nucléotides,
   ∘ E1 représente un espaceur qui bloque la polymérisation du brin complémentaire à ladite séquence nucléotidique T1 par une ADN polymérase dépourvue d'activité exonucléasique et possédant la propriété de déplacement du brin,
   ∘ A1 représente une séquence nucléotidique, de préférence constituée de 10 à 40 nucléotides, de préférence de 15 à 25 nucléotides, apte à s'hybrider audit ADN monocaténaire circularisé et auxdits brins négatifs ; par conséquent, la séquence nucléotidique de A1 étant complémentaire à un fragment dudit ADN monocaténaire circularisé, et
   ∘ n1 et m1 sont indépendamment un nombre entier égal à 0 ou 1, de préférence n1 = 0 et m1 = 1 ;
   et/ou
- ladite amorce anti-sens est constituée de ou comprend, de son extrémité 5' vers son extrémité 3', la séquence suivante : 5'-(F2)ₙ₂-T2-(E2)ₘ₂-A2-3', dans laquelle :
   ∘ F2 représente un groupement terminal, choisi parmi un marqueur et un agent de couplage, qui peut être identique au ou différent du groupement terminal F1,
   ∘ T2 représente une séquence nucléotidique code barre, de préférence constituée de 6 à 30 nucléotides, de préférence de 10 à 25 nucléotides, qui peut être identique à ou différente de la séquence nucléotidique T1,
   ∘ E2 représente un espaceur qui bloque la polymérisation du brin complémentaire à ladite séquence nucléotidique T2 par une ADN polymérase dépourvue d'activité exonucléasique et possédant la propriété de déplacement du brin,
   ∘ A2 représente une séquence nucléotidique, de préférence constituée de 10 à 40 nucléotides, de préférence de 15 à 25 nucléotides, apte à s'hybrider auxdits brins positifs ; par conséquent, la séquence nucléotidique de A2 étant identique à un fragment dudit ADN monocaténaire circularisé, et
   ∘ n2 et m2 sont indépendamment un nombre entier égal à 0 ou 1, de préférence n2 = 1 et m2 = 0 ;
   ledit ADN monocaténaire circularisé étant détecté par hybridation desdites séquences codes barres T1 et/ou T2 présentes aux extrémités desdits ADN périodiques bicaténaires à une sonde nucléotidique complémentaire auxdites séquences codes barres T1 et/ou T2.

Lorsqu'une amorce sens de séquence 5'-(F1)ₙ₁-T1-(E1)ₘ₁-A1-3' ou une amorce antisens de séquence 5'-(F2)ₙ₂-T2-(E2)ₘ₂-A2-3' telles que définies ci-dessus est utilisée pour mettre en oeuvre le procédé de détection selon la présente description, alors l'autre amorce (respectivement antisens ou sens) est tout type d'amorce utilisable dans le cadre d'une amplification ramifiée isotherme par cercle roulant.

L'amplification ramifiée isotherme par cercle roulant en utilisant les amorces telles que définies ci-dessus sera appelée ci-après THRCA (« *tagged hyperbranched rolling circle amplification* »). La THRCA s'effectue à une température comprise entre 37°C et 70°C, de préférence entre 60°C et 65°C.

La présente invention a pour objet un procédé de détection d'un ADN monocaténaire circularisé par amplification ramifiée isotherme par cercle roulant (HRCA) dudit ADN monocaténaire circularisé en présence d'une amorce sens apte à s'hybrider audit ADN monocaténaire circularisé et aux brins négatifs générés lors de la HRCA et d'une amorce anti-sens apte à s'hybrider aux brins positifs générés lors de la HRCA, ladite HRCA générant des ADN bicaténaires périodiques, lequel procédé étant caractérisé en ce que :
- ladite amorce sens est constituée de, ou comprend, de son extrémité 5' vers son extrémité 3', la séquence suivante : 5'-(F1)ₙ₁-T1-E1-A1-3' dans laquelle :
   ∘ F1 représente un groupement terminal choisi parmi un marqueur et un agent de couplage ;
   ∘ T1 représente une séquence nucléotidique code-barre constituée de 6 à 30 nucléotides ;
   ∘ El représente un espaceur qui bloque la polymérisation du brin complémentaire à ladite séquence nucléotidique T1 par une ADN polymérase dépourvue d'activité exonucléasique et possédant une activité de déplacement de brin ;
   ∘ A1 représente une séquence nucléotidique, constituée de 10 à 40 nucléotides, apte à s'hybrider audit ADN monocaténaire circularisé et auxdits brins négatifs ; et
   ∘ n1 est un nombre entier égal à 0 ou 1 ;
   et/ou
- ladite amorce anti-sens est constituée de, ou comprend, de son extrémité 5' vers son extrémité 3', la séquence suivante : 5'-(F2)ₙ₂-T2-E2-A2-3', dans laquelle :
   ∘ F2 représente un groupement terminal, choisi parmi un marqueur et un agent de couplage, qui peut être identique au ou différent du groupement terminal F1 ;
   ∘ T2 représente une séquence nucléotidique code-barre, constituée de 6 à 30 nucléotides, qui peut être identique à ou différente de la séquence nucléotidique T1 ;
   ∘ E2 représente un espaceur qui bloque la polymérisation du brin complémentaire à ladite séquence nucléotidique T2 par une ADN polymérase dépourvue d'activité exonucléasique et possédant une activité de déplacement de brin, et qui peut être identique à ou différent de l'espaceur El ;
   ∘ A2 représente une séquence nucléotidique, constituée de 10 à 40 nucléotides, apte à s'hybrider auxdits brins positifs ; et
   ∘ n2 est un nombre entier égal à 0 ou 1 ;
   ledit ADN monocaténaire circularisé étant détecté par hybridation desdites séquences codes-barres T1 et/ou T2 présentes aux extrémités desdits acides nucléiques périodiques bicaténaires à une sonde nucléotidique complémentaire auxdites séquences codes-barres T1 et/ou T2, où ladite sonde nucléotidique est présente sur un support solide.

Selon un autre aspect, la présente invention a pour objet un procédé de détection d'un polymorphisme génétique d'une seule ou de plusieurs paire(s) de bases, comprenant les étapes suivantes :
i) mise en contact d'un acide nucléique cible susceptible de contenir une ou plusieurs bases polymorphes à détecter avec un ADN monocaténaire circularisable (sonde) ;
ii) hybridation dudit ADN monocaténaire à l'acide nucléique cible ;
iii) obtention, si l'acide nucléique cible contient la ou lesdites bases polymorphes, d'un ADN monocaténaire hybridé et circularisé par ligation dudit ADN monocaténaire hybridé à l'acide nucléique cible en présence d'une ADN ligase ;
iv) obtention d'ADN bicaténaires périodiques par amplification ramifiée isotherme par cercle roulant dudit ADN monocaténaire hybridé et circularisé, en présence d'au moins une des deux amorces de séquences 5'-(F1)ₙ₁-T1-E1-A1-3' et 5'-F2)ₙ₂-T2-E2-A2-3' telles que définies ici ;
v) détection des séquences codes-barres T1 et/ou T2 présentes aux extrémités des acides nucléiques périodiques bicaténaires obtenus à l'étape iv) par hybridation à une sonde nucléotidique complémentaire auxdites séquences codes-barres T1 et/ou T2, où ladite sonde nucléotidique est présente sur un support solide ; la détection des séquences codes-barres T1 et/ou T2 présentes aux extrémités desdits ADN périodiques bicaténaires indiquant que ledit acide nucléique cible contient la ou lesdites bases polymorphes.

Selon encore un autre aspect, la présente invention a pour objet l'utilisation d'au moins une paire d'amorces sens et anti-sens pour la détection d'un ADN monocaténaire circularisé par amplification ramifiée isotherme par cercle roulant (HRCA) ; dans laquelle l'amorce sens est apte à s'hybrider audit ADN monocaténaire circularisé et aux brins négatifs générés lors de la HRCA, et l'amorce anti-sens est apte à s'hybrider aux brins positifs générés lors de la HRCA ; et dans laquelle ladite amorce sens est constituée de, ou comprend, de son extrémité 5' vers son extrémité 3', la séquence suivante : 5'-(F1)ₙ₁-T1-E1-A1-3', telle que définies ici et/ou ladite amorce anti-sens est constituée de, ou comprend, de son extrémité 5' vers son extrémité 3', la séquence suivante : 5'-(F2)ₙ₂-T2-E2-A2-3', telle que définies ici ; et d'un support solide comprenant des séquences nucléotidiques qui sont complémentaires à la séquence code-barre.

Selon un autre aspect, la présente invention a pour objet un kit ou coffret de détection d'un polymorphisme génétique d'une seule ou de plusieurs paire(s) de bases, caractérisé en ce qu'il comprend au moins une paire d'amorces sens et anti-sens dans laquelle ladite amorce sens est constituée de, ou comprend, de son extrémité 5' vers son extrémité 3', la séquence suivante : 5'-(F1)ₙ₁-T1-E1-A1-3', telle que définie à l'une quelconque des revendications 1 à 5 et/ou ladite amorce anti-sens est constituée de, ou comprend, de son extrémité 5' vers son extrémité 3', la séquence suivante : 5'-(F2)ₙ₂-T2-E2-A2-3', telle que définies ici, un ADN monocaténaire circularisable, une ADN ligase, une ADN polymérase dépourvue d'activité exonucléasique et possédant une activité de déplacement de brin, des désoxynucléotides, un tampon approprié et une puce à ADN ; où ladite puce à ADN porte des séquences nucléotidiques qui sont complémentaires à la séquence code-barre.

On entend par bloquer la polymérisation du brin complémentaire, le fait qu'une ADN polymérase, telle qu'une ADN polymérase dépourvue d'activité exonucléasique et possédant la propriété de déplacement du brin, ne continue pas la synthèse (polymérisation) du brin complémentaire à la séquence nucléotidique T (T1 ou T2), lorsqu'elle arrive au niveau de l'espaceur E (E1 ou E2) après avoir synthétisé le brin complémentaire de la séquence nucléotidique A (A1 ou A2). La mise en évidence de la fonction de blocage de la polymérisation au cours de la THRCA par un espaceur peut être effectuée conformément à l'Exemple 1 ci-dessous (voir le paragraphe 1-2-a).

Selon un mode de mise oeuvre préféré de l'invention, l'ADN polymérase dépourvue d'activité exonucléasique et possédant la propriété de déplacement du brin est l'ADN polymérase *Bst* Grand Fragment ou l'ADN polymérase *Vent* exo-.

Selon un autre mode de mise oeuvre préféré de la description, m1 + m2 est égal à 1 ou 2 ; c'est-à-dire qu'une ou les deux amorces comprennent un espaceur.

L'espaceur E1 et/ou E2 est de préférence choisi dans le groupe constitué par un site abasique, un groupe alkyle, alcényle ou alcynyle, linéaire ou ramifié, éventuellement substitué. De tels espaceurs sont bien connus de l'homme du métier (voir par exemple la Demande de Brevet EP 0 416 817).

A titre d'exemple avantageux d'espaceurs, on peut citer les espaceurs constitués de ou comprenant un polyéthylène glycol, constitué d'un enchaînement de 1 à 100, de préférence de 1 à 50, de préférence encore de 2 à 10, de préférence encore de 4 à 8 et plus préférentiellement de 6 unités éthylène glycol, et d'un groupe PO₄ à l'une de leurs extrémités.

De préférence, l'espaceur E1 et/ou E2 est de formule I : 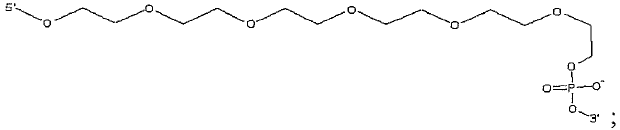 la numérotation 5' et 3' sur la formule I indiquant que l'« extrémité 5' » de l'espaceur est liée à l'extrémité 3' de la séquence T (T1 ou T2) et que l'« extrémité 3' » est liée à l'extrémité 5' de la séquence A (A1 ou A2).

Les méthodes de synthèse d'oligonucléotides comprenant un espaceur tel que défini ci-dessus sont bien connues de l'homme du métier.

Selon un mode de mise en oeuvre avantageux de l'invention, les séquences T1, A1, T2 et A2 ne s'hybrident pas entre elles dans les conditions réactionnelles de la THRCA.

Le groupement terminal est un composé couplé à l'extrémité 5' de la séquence code barre T (T1 ou T2), qui peut être un marqueur, utilisé pour la détection de l'ADN, ou un agent de couplage. Il est choisi de préférence dans le groupe constitué par :
- un agent luminescent, tel que les agents radioluminescents, chimioluminescents, fluorescents (par exemple Cy3 et Cy5) ou phosphorescents, ou une boîte quantique (*quantum dot*),
- un radioisotope, tel que le ³²P,
- une enzyme, telle qu'une enzyme ayant un substrat chromogène, fluorigène ou luminescent (par exemple une peroxydase ou une phosphatase alcaline) ou des enzymes produisant ou utilisant des protons (oxydase ou hydrolase),
- un groupe acrylamide (Li et al., 2009, Clinica Chimica Acta, 399, 40-44),
- la biotine,
- un thiol, tel qu'un 5'-thiol modificateur C6, ou un 5'-thiol modificateur C6 S-S, et
- un phosphorothioate.

La séquence code barre T (T1 et T2) présente dans les amorces est monocaténaire. Elle est de préférence constituée de 10 à 22 nucléotides, de préférence encore de 20 nucléotides. Elle n'est pas apte à s'hybrider audit ADN monocaténaire circularisé ni auxdits brins négatifs et positifs, dans les conditions réactionnelles de la THRCA. Sa séquence peut être choisie de différentes manières par l'homme du métier, notamment à l'aide d'un programme d'ordinateur afin d'obtenir une reconnaissance spécifique par hybridation (absence d'hybridation parasite entre séquences codes barres, température de fusion similaires).

La séquence nucléotidique A (A1 et A2) est de préférence constituée de 20 nucléotides. Sa séquence est fonction de la séquence dudit ADN monocaténaire circularisé, et peut être choisie de différentes manières par l'homme du métier, notamment à l'aide d'un programme d'ordinateur. Les températures de fusion de A1 et A2 sont de préférence similaires, et sont avantageusement comprises entre 50 et 60°C inclus, à un pH compris entre 7 et 9 inclus.

Avantageusement, les séquences A1 et A2 ne sont pas complémentaires, afin d'éviter leur dimérisation.

Selon un autre mode de mise en oeuvre de la description, dans le cas où m1 + m2 est égal à 0 ou 1 (c'est-à-dire au moins une des deux amorces ne comprend pas d'espaceur), de préférence égal à 0, alors avant l'étape de détection des séquences codes barres, lesdits ADN périodiques bicaténaires obtenus par la THRCA peuvent être digérés par une enzyme de manière à rendre les séquences codes barres monocaténaires.

Des méthodes de digestion d'un ADN bicaténaire par une enzyme de manière à obtenir un ADN bicaténaire dont l'une ou les deux extrémités est/sont monocaténaire(s) sont connues de l'homme du métier. On peut, par exemple, chauffer l'ADN bicaténaire et utiliser une exonucléase, ou bien utiliser une enzyme de restriction ou une polymérase qui reconnait une séquence promoteur (Sambrook et Russell, 2001, Molecular Cloning: A Laboratory Manual, 3rd Ed. Cold Spring Harbor Laboratory Press, Cold Spring Harbor, NY). On peut également utiliser des didésoxyribonucléotides (ddNTP) pour la polymérisation et ensuite utiliser une enzyme de restriction qui ne pourra couper qu'un seul brin en raison de la présence des ddNTPs.

Avantageusement, lorsque l'on utilise une exonucléase pour obtenir un ADN bicaténaire ayant une extrémité mono caténaire, alors les brins d'ADN qui ne doivent pas être digérés par l'exonucléase peuvent être protégés par une modification terminale (par exemple par ajout d'un phosphorothioate) pour empêcher l'exonucléase d'agir sur ce brin d'ADN.

La détection de la séquence code barre monocaténaire à l'extrémité des ADN bicaténaires obtenues en mettant en oeuvre la THRCA peut être effectuée de différentes manières connues de l'homme du métier, par exemple par détection de son hybridation à une séquence nucléotidique (sonde) complémentaire à ladite séquence code barre, fixée sur un support solide, tel qu'une puce à ADN (*microarray*). Les méthodes de détection d'une séquence hybridée à une sonde fixée à un support sont aussi bien connues de l'homme du métier (Sambrook et Russell, 2001, Molecular Cloning: A Laboratory Manual, 3rd Ed. Cold Spring Harbor Laboratory Press, Cold Spring Harbor, NY). Elle peut se faire, par exemple, soit par détection du marqueur F1 ou F2, soit par une technique de détection par hybridation dans laquelle le signal de détection de l'hybridation entre la séquence code barre et la séquence nucléotidique (sonde) complémentaire à ladite séquence code barre augmente avec le nombre de nucléotides de la molécule d'acide nucléique comprenant ladite séquence code barre, telle que la résonance plasmonique de surface (*e.g*., du type Biacore), la mesure de l'absorption UV, la microscopie à force atomique (AFM), la détection utilisant une puce comprenant un réseau de poutres (« mechanical cantilever arrays ; Fritz et al., 2000, Science 288, 316-318), ou la détection électronique à l'aide de transistors à effet de champ (FET ou « *Field Effect Transistor* »)).

Le support solide servant à fixer ladite sonde peut être un matériau plastique (polystyrène, polycarbonate par exemple), ou bien du nylon ou du verre.

Les méthodes de détection de l'hybridation d'un acide nucléique à un support solide (par l'intermédiaire d'une sonde nucléotidique complémentaire audit acide nucléique), dans lesquelles le signal augmente en fonction de la taille des molécules hybridées, sont particulièrement avantageuses pour la détection d'une séquence code barre monocaténaire à l'extrémité des ADN bicaténaires obtenues en mettant en oeuvre la THRCA selon la présente description. En effet, les produits d'amplification obtenus sont de grande taille, ce qui permet leur bonne détection par une technique de détection par hybridation dans laquelle le signal de détection de l'hybridation entre la séquence code barre et une séquence nucléotidique (sonde) complémentaire à ladite séquence code barre augmente avec le nombre de nucléotides, telle que par exemple une méthode de détection électronique.

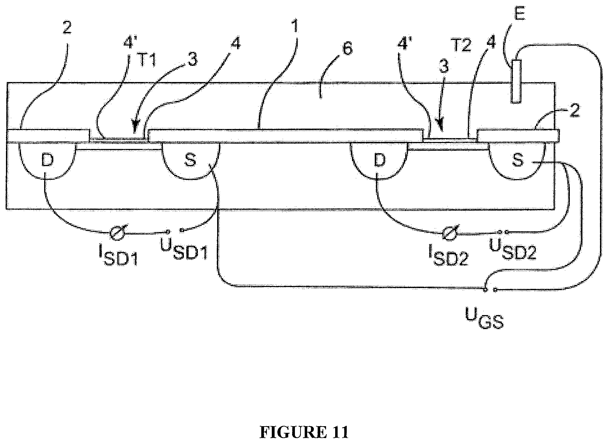

A titre d'exemple non limitatif de dispositif pour effectuer une détection électronique, on peut citer un capteur présentant un réseau de transistors à effet de champ (FET) sur un substrat en silicium. Un transistor T₁ ou T₂ représenté en coupe à la Figure 11 est pourvu d'une région de source S et d'une région de drain D qui présentent chacune un contact électrique et qui sont surmontées d'une couche isolante respectivement 1 et 2, par exemple un oxyde thermique de Si0₂. La région active 3 entre la source S et le drain D forme la région de porte G du transistor et présente une couche isolante 4 d'épaisseur réduite (de 2 nm à 50 nm, de préférence 10 nm), par exemple une couche de Si0₂ thermique.

Une sonde nucléotidique simple brin (*e.g*., ADN) complémentaire de ladite séquence nucléotidique code barre est fixée par un procédé connu de l'homme du métier, sur au moins certaines des surfaces actives 4. On utilise de préférence des transistors à effet de champ à canal n à appauvrissement (pour lesquels les porteurs de charge sont les électrons, plus mobiles d'où une augmentation de la sensibilité de la détection) avec une polarisation de porte négative (c'est-à-dire que l'électrolyte 6 est polarisé négativement par rapport au semi-conducteur), la sonde se chargeant négativement (pour un électrolyte de pH neutre).

L'application d'une tension source-drain U_{SD} entre la source S et le drain D (U_{SD1} pour T₁ et U_{SD2} pour T₂) et d'une tension porte-source U_{GS} entre l'électrolyte 6 et la source S (par exemple par une électrode E unique Ag/AgCl) induit un gaz bidimensionnel de porteurs de charges à l'interface Si/Si0₂, ou à l'interface Si/électrolyte de chaque transistor. Il en résulte un courant de drain I_{D} qui, pour chaque transistor, dépend de façon sensible de la charge à l'interface SiO₂/électrolyte ou Si/électrolyte. On appelle surface active cette interface qui fait face au canal entre la source S et le drain D.

Le courant I_{D} dépend de la fixation des sondes sur la surface active 4.

On peut réaliser une détection à tension source-porte U_{SG} et à tension source-drain U_{SD} constants, en mesurant le courant de drain I_{Sd} ou bien encore, à courant de drain I_{Sd} et à tension source-drain U_{SD} constants, en mesurant la tension source-porte U_{SG}.

Un tel dispositif est décrit dans la Demande Internationale WO 2004/057027.

La détection électronique mettant en oeuvre un capteur (tel que décrit ci-dessus) comprenant un réseau de transistors à effet de champ (T₁, T₂,...) dont chacun présente une région de source (5), une région de drain (D), ainsi qu'une région de porte qui constitue une zone active (3) sur laquelle l'hybridation spécifique entre ladite sonde nucléotidique et ladite séquence nucléotidique code barre doit être détectée, peut comprendre les étapes suivantes :
a) fournir un capteur comprenant au moins deux zones actives (3) (voir Figure 11) sur lesquelles sont fixées des sondes nucléotidiques complémentaires des séquences codes barres,
b) mettre en contact lesdites sondes nucléotidiques avec le produit d'amplification obtenu par la méthode THRCA selon la présente description dans un tampon réactionnel ayant une première concentration en sel pour obtenir une hybridation spécifique entre lesdites sondes nucléotidiques et les séquences codes barres ; et
c) dans un tampon de mesure ayant une deuxième concentration en sel qui est inférieure à celle du tampon réactionnel, mesurer au moins un point de la caractéristique courant de drain/tension source-porte/tension source-drain d'au moins un transistor dudit réseau pour détecter ladite hybridation spécifique.

Avantageusement, la mesure est effectuée de manière différentielle pour des sondes fixées sur des zones actives (3) distinctes. Cette mesure différentielle est obtenue, selon une première variante, par différence entre le point de mesure et un point de référence ; le point de mesure étant obtenu pour des sondes nucléotidiques ayant été hybridées à une séquence nucléotidique code barre (telle que définie ci-dessus) à l'étape b) et le point de référence étant obtenu pour des sondes nucléotidiques n'ayant pas été hybridées. Selon une deuxième variante, la mesure différentielle est obtenue par différence entre deux points de mesure obtenus pour des sondes fixées sur des zones actives (3) distinctes et soumises à deux hybridations différentes, une première hybridation spécifique avec ladite séquence nucléotidique code barre et une deuxième interaction avec une autre séquence nucléotidique différente de ladite séquence nucléotidique code barre.

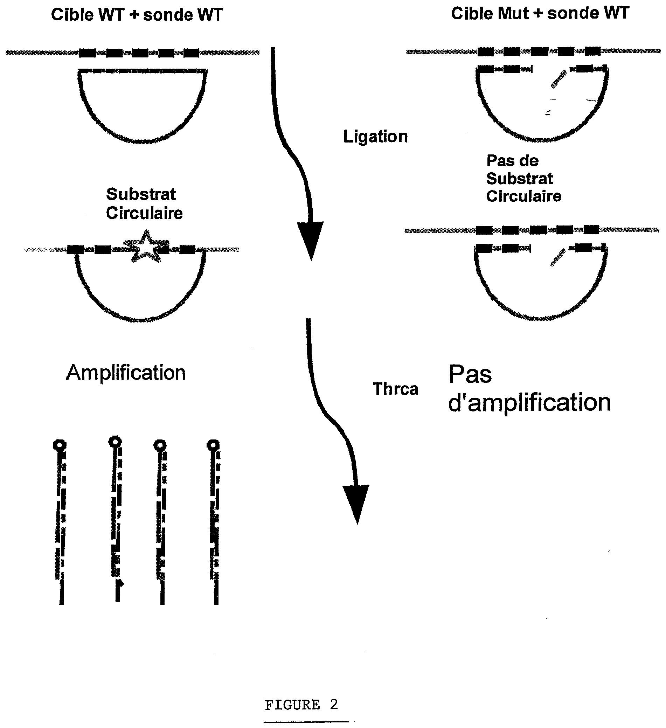

La THRCA peut être utilisée pour la détection d'un polymorphisme génétique d'une seule ou de plusieurs paire(s) de base (mutations). Le principe de détection d'un polymorphisme génétique par THRCA est représenté à la Figure 2. Dans le cas d'une détection d'un polymorphisme génétique par multiplexage (détection de plusieurs mutations) et/ou en parallèle (détection réalisée par exemple sur différents patients à l'aide d'une puce à ADN), alors la séquence code barre peut être associée à une mutation particulière ou à un patient particulier.

La présente description donc également pour objet un procédé de détection d'un polymorphisme génétique d'une seule ou de plusieurs paire(s) de base (substitution, insertion ou délétion d'une ou plusieurs bases), comprenant les étapes suivantes :
i) mise en contact d'un acide nucléique cible susceptible de contenir une ou plusieurs bases polymorphes à détecter avec un ADN monocaténaire circularisable (sonde) spécifique de la ou desdites bases polymorphes,
ii) hybridation dudit ADN monocaténaire à l'acide nucléique cible,
iii) obtention d'un ADN monocaténaire hybridé et circularisé si l'acide nucléique cible contient la ou lesdites bases polymorphes, par ligation dudit ADN monocaténaire hybridé à l'acide nucléique cible en présence d'une ADN ligase (*e.g.* T4 ADN ligase), de préférence une ADN ligase thermostable, telle que l'ADN ligase Ampligase (Epicentre Technologies).
iv) obtention d'ADN bicaténaires périodiques par amplification ramifiée isotherme par cercle roulant dudit ADN monocaténaire hybridé et circularisé, en présence d'au moins une des deux amorces de séquences 5'-(F1)ₙ₁-Tl-(E1)ₘ₁-A1-3' et 5'-(F2)ₙ₂-T2-(E2)ₘ₂-A2-3' telles que définies ci-dessus, m1 + m2 étant de préférence égal à 1 ou 2,
v) détection des séquences codes barres T1 et/ou T2 présentes aux extrémités des ADN périodiques bicaténaires obtenus à l'étape iv) par hybridation à une sonde nucléotidique complémentaire auxdites séquences codes barres T1 et/ou T2, comme indiqué ci-dessus ; la détection des séquences codes barres T1 et/ou T2 présentes aux extrémités desdits ADN périodiques bicaténaires indiquant que ledit acide nucléique cible contient une ou plusieurs bases polymorphes.

Bien entendu, selon ce mode de mise en oeuvre de la description, la séquence nucléotidique A1 n'est pas complémentaire au fragment de la séquence dudit ADN monocaténaire circularisé qui est hybridé à l'acide nucléique cible.

A l'étape iv), le temps d'incubation optimal pour l'amplification ramifiée isotherme par cercle roulant peut être déterminé par des essais de routine par l'homme du métier. Généralement, on préfère un temps de polymérisation le plus court possible pour éviter d'amplifier les sondes circularisables linéaires présentes, mais suffisamment long pour obtenir lesdits ADN périodiques bicaténaires en quantité détectable. De préférence, le temps d'incubation à l'étape iv) est de 25 à 90 minutes, de préférence encore de 25 à 35 minutes.

Dans le cadre d'une détection d'un polymorphisme génétique par multiplexage, c'est-à-dire une détection de plusieurs mutations (une mutation étant définie comme une ou plusieurs bases polymorphes) à une position donnée sur un ADN cible, l'ADN cible est mis en contact, à l'étape i) ci-dessus, avec plusieurs ADN monocaténaires circularisables (sondes) différents ; chaque ADN monocaténaire circularisable (sonde) étant spécifique d'une mutation donnée à ladite position. A l'étape iii) ci-dessus, l'ADN monocaténaire circularisé et hybridé à l'acide nucléique cible est alors celui qui est spécifique d'une mutation donnée. A l'étape iv) ci-dessus, plusieurs amorces de séquences 5'-(F1)ₙ₁-T1-(E1)ₘ₁-A1-3' et/ou 5'-(F2)ₙ₂-T2-(E2)ₘ₂-A2-3' telles que définies ci-dessus sont utilisés ; les séquences nucléotidiques A1 et A2, et les séquences nucléotidiques T1 et/ou T2 étant spécifiques d'un ADN monocaténaire circularisable (sonde) donné. A l'étape v), la détection des séquences codes barres T1 et/ou T2 présentes aux extrémités des ADN périodiques bicaténaires obtenus à l'étape iv), indique que ledit acide nucléique cible contient une mutation donnée.

On entend par acide nucléique cible, une séquence d'ADN (tel que de l'ADN génomique) ou d'ARN monocaténaire ou bicaténaire. De préférence, cet acide nucléique cible est de l'ADN.

Dans le cas où l'acide nucléique cible est bicaténaire, alors une étape de dénaturation peut être effectuée avant l'étape i) de manière à rendre cet acide nucléique bicaténaire sous une forme monocaténaire.

Avant l'étape i), il peut aussi être nécessaire de fractionner l'acide nucléique cible en mettant en contact ledit acide nucléique cible avec une ou plusieurs enzymes de restriction bien connues de l'homme du métier. A titre d'exemples non limitatifs enzymes de restriction, on peut utiliser les enzymes ou cocktails d'enzymes suivants :
- *Alu*I*,*
- *Dra*I et *Ssp*I
- *EcoR*I*, Hind*III et *BamH*I, et
- *Ase*I*.*

Selon cet aspect de la description, ledit ADN monocaténaire circularisable est aussi appelé sonde. Elle est constituée d'une séquence d'ADN monocaténaire linéaire, de 30 à 140 nucléotides, de préférence 50 à 100 nucléotides, dont les extrémités, d'environ 20 nucléotides chacune, sont aptes à s'hybrider (c'est-à-dire sont complémentaires) à des fragments contigus de l'acide nucléique cible, de façon à ce que les extrémités 5' et 3' dudit ADN monocaténaire soient adjacentes. Lorsque les extrémités dudit ADN monocaténaire sont hybridées à l'acide nucléique cible alors elles peuvent être liées ensemble par une ADN ligase pour former un ADN monocaténaire circularisé. La séquence nucléotidique entre les extrémités 5' et 3' de l'ADN monocaténaire contient un fragment complémentaire de l'amorce sens telle que définie ci-dessus. Un tel ADN monocaténaire circularisable (sonde) est bien connu de l'homme du métier.

Selon un mode de mise en oeuvre avantageux de cet aspect de la description, les étapes i) à iv) sont réalisées par mélange dudit ADN monocaténaire circularisable (sonde), ledit acide nucléique cible monocaténaire, lesdites amorces, des désoxynucléotides (dNTP), une ADN ligase, des rATP, une ADN polymérase dépourvue d'activité exonucléasique et possédant la propriété de déplacement du brin, et un stabilisateur de ladite polymérase (tel que l'albumine sérique bovine (BSA) ou du Triton X-100), dans un tampon approprié dans lequel ladite ADN ligase circularise l'ADN monocaténaire hybridé à l'ADN cible si l'hybridation ne contient aucun mésappariement, et ladite ADN polymérase catalyse la polymérisation des brins d'ADN.

Selon ce mode de mise en oeuvre, la ligation et la polymérisation s'effectuent dans le même tampon. Il n'est donc pas nécessaire de changer de milieu réactionnel, d'ajouter des réactifs ou de réaliser une purification du produit de ligation pour permettre la réaction d'amplification par THRCA.

De préférence, ledit tampon comprend ou est constitué d'acétate de potassium, de tris-acétate, d'acétate de magnésium et de dithiothréitol, de préférence à un pH compris entre 7 et 9 inclus, de préférence encore compris entre 7,8 et 8,5 inclus.

De préférence encore, ledit tampon comprend ou est constitué de 50 mM d'acétate de potassium, 20 mM de tris-acétate, 10 mM d'acétate de magnésium, et 1 mM de dithiothréitol, de préférence à un pH de 7,9.

Selon une disposition avantageuse de ce mode de mise en oeuvre, la ligation de la sonde circularisable s'effectue à une température comprise entre 1°C et 37°C de préférence entre 16°C et 25°C (températures inférieures à la température d'amplification).

Selon une autre une disposition avantageuse de ce mode de mise en oeuvre, ledit mélange ne contient pas ladite ADN polymérase et/ou lesdites amorces, qui sont ajoutées une fois la ligation de la sonde circularisable effectuée.

La présente description a aussi pour objet l'utilisation d'au moins une amorce constituée de ou comprenant, de son extrémité 5' vers son extrémité 3', la séquence suivante : 5'-(F)ₙ-T-E-A-3', dans laquelle
∘ F représente un groupement terminal tel que défini ci-dessus,
∘ T représente une séquence nucléotidique code barre, de préférence constituée de 6 à 30 nucléotides, de préférence de 10 à 25 nucléotides, telle que définie ci-dessus,
∘ E représente un espaceur tel que défini ci-dessus qui bloque la polymérisation du brin complémentaire à ladite séquence nucléotidique T par une ADN polymérase, de préférence une ADN polymérase dépourvue d'activité exonucléasique et possédant la propriété de déplacement du brin,
∘ A représente une séquence nucléotidique, de préférence constituée de 10 à 40 nucléotides, de préférence de 15 à 25 nucléotides, apte à s'hybrider à un acide nucléique cible (ou substrat) et à initier la polymérisation du brin complémentaire dudit acide nucléique cible par ladite ADN polymérase, de préférence une ADN polymérase dépourvue d'activité exonucléasique et possédant la propriété de déplacement du brin, et
∘ n est un nombre entier égal à 0 ou 1, de préférence égal à 1, pour l'amplification d'une séquence d'ADN par la technique PCR (par exemple par PCR multiplexe, RAPD-PCR, AS-PCR (PCR allèle spécifique)) ou la détection d'un ADN monocaténaire circularisé par amplification ramifiée isotherme par cercle roulant ou la détection d'un polymorphisme génétique d'une seule ou de plusieurs paire(s) de bases.

La présente description a également pour objet un kit ou coffret de détection d'un ADN monocaténaire circularisé ou d'un polymorphisme génétique d'une seule ou de plusieurs paire(s) de bases, caractérisé en ce qu'il comprend au moins une paire d'amorces sens et anti-sens de séquences 5'-(F1)ₙ₁-T1-(E1)ₘ₁-A1-3' et 5'-(F2)ₙ₂-T2-(E2)ₘ₂-A2-3' telles que définies ci-dessus, une ADN ligase, une ADN polymérase dépourvue d'activité exonucléasique et possédant la propriété de déplacement du brin, des désoxynucléotides, un tampon approprié, une puce à ADN, et, pour la détection d'un polymorphisme génétique, un ADN monocaténaire circularisable (sonde).

D'autres aspects et avantages de la présente invention apparaîtront à la lecture des exemples qui suivent, qui doivent être considérés comme illustratifs et non limitatifs, ainsi que des figures annexées :
**Figure 1** : représentation schématique du principe de la THRCA : exemple avec un ADN monocaténaire (sonde) circularisé et hybridé à un acide nucléique cible monocaténaire et un couple d'amorces selon la présente description dont une amorce sens comprenant un espaceur mais pas de fluorophore et une amorce anti-sens comprenant un fluorophore mais pas d'espaceur ; **A** : les oligonucléotides entrant en jeu ; (1) : acide nucléique cible monocaténaire (par exemple ADN génomique) ; (2) : ADN monocaténaire linéaire circularisable (sonde) ; (3) : ADN monocaténaire (sonde) circularisé (brin négatif) ; (4) : amorce anti-sens, constituée de son extrémité 5' vers son extrémité 3', d'un fluorophore, d'une séquence nucléotidique code barre (appelée aussi séquence de liaison) et d'une séquence nucléotidique identique à un fragment de ladite sonde ; (5) amorce sens, constituée de son extrémité 5' vers son extrémité 3' d'une séquence nucléotidique code barre, d'un espaceur et d'une séquence nucléotidique complémentaire à un fragment de ladite sonde ; (6) : zone d'hybridation de ladite sonde sur l'acide nucléique cible ; (7) : fluorophore ; (8) : espaceur ; (9) : séquence nucléotidique code barre ; **B** : formation du substrat circulaire ; l'étoile représente la ligase qui permet la ligation de la sonde circularisée hybridée à l'acide nucléique cible ; **C** : amplification par THRCA ; (10) : hybridation de l'amorce sens sur le substrat circulaire (brin négatif); (11): polymérisation du brin complémentaire (brin positif) à la sonde (l'ADN polymérase est représentée par un hexagone) ; (12) : lorsqu'elle rencontre l'ADN cible, la polymérase déplace le brin et poursuit sa synthèse (polymérisation du brin complémentaire à la sonde) ; (13) : obtention d'un ADN monocaténaire périodique (brin positif) ; (14) ; hybridation des amorces anti-sens à l'ADN monocaténaire périodique et synthèse (polymérisation) des brins complémentaires (brins négatifs) ; le déplacement des brins d'ADN synthétisés va permettre la synthèse d'ADN monocaténaires périodiques de différentes tailles (brin négatifs) ; (15) : obtention de plusieurs brins monocaténaires périodiques comprenant à l'une de leurs extrémités la séquence code barre (séquence de liaison) et le fluorophore ; (16) : hybridation de l'amorce sens sur le brin monocaténaire périodique et synthèse (polymérisation) du brin complémentaire ; (17) : obtention de brins bicaténaires périodiques marqués par un fluorophore à l'une de leurs extrémités et par une séquence code barre monocaténaire à l'autre extrémité.
**Figure 2** : principe de détection d'un polymorphisme génétique par THRCA. Cible WT = ADN génomique (par exemple allèle) de type sauvage ; Cible Mut = ADN génomique (par exemple allèle) contenant une mutation d'une seule ou de plusieurs paire(s) de bases ; sonde WT : sonde monocaténaire circularisable apte à être circularisée et hybridée audit ADN génomique de type sauvage mais pas audit ADN génomique contenant une mutation. Les légendes sont identiques à celles de la Figure 1.

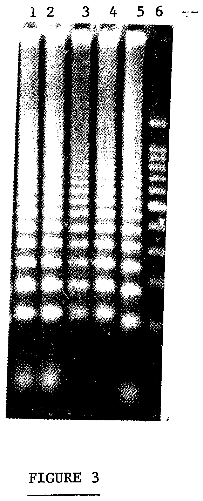

**Figure 3** : photographie de gel mettant en évidence le blocage de la polymérase au cours de la THRCA. Puits 1 et 2 : produits d'amplification par THRCA du substrat ADN cible de type sauvage / sonde WTCirc avec le couple d'amorces WTFP non marquée et WTRP marquée au Cy3 ; Puits 3 et 4 : produits d'amplification par THRCA du substrat ADN cible de type sauvage / sonde WTCirc avec le couple d'amorces WTFP non marquée et WTRP marquée au Cy5 ; Puits 5 : produits d'amplification par THRCA du substrat ADN cible de type sauvage / sonde WTCirc avec le couple d'amorces WTFP ne contenant pas d'espaceur et WTRP non marquée. Puits 6 : marqueur de poids moléculaire 100bp NEB.

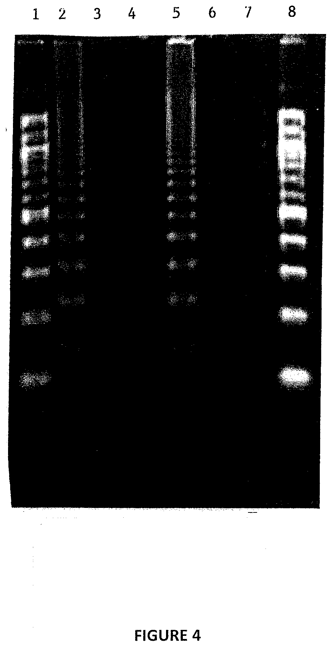

**Figure 4** : photographie de gel mettant en évidence la spécificité de l'amplification par THRCA. Puits 1 et 8 : marqueur de poids moléculaire 100bp NEB. Puits 2 : produit d'amplification du substrat A (ADN cible de type mutant / sonde MutCirc) obtenu après 30 min de réaction THRCA ; Puits 3 : produit d'amplification du substrat B (ADN cible de type mutant / sonde WTCirc) obtenu après 30 min de réaction THRCA. Puits 4 : produit d'amplification du substrat C (ADN cible de type sauvage / sonde MutCirc) obtenu après 30 min de réaction THRCA ; Puits 5 : produit d'amplification du substrat A obtenu après 40 min de réaction THRCA ; Puits 6 : produit d'amplification du substrat B obtenu après 40 min de réaction THRCA ; Puits 7 : produit d'amplification du substrat C obtenu après 40 min de réaction THRCA.

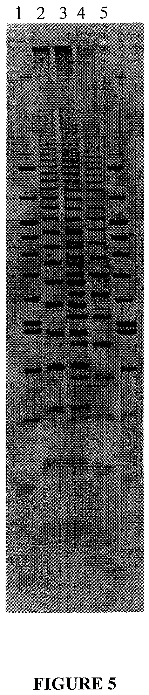

**Figure 5** : photographie de gel mettant en évidence l'amplification par THRCA avec un multiplexage. Puits 1 : marqueur de poids moléculaire 100bp NEB ; Puits 2 : produit d'amplification par THRCA du substrat A ; Puits 3 : produit d'amplification par THRCA des substrats A (ADN cible de type mutant / sonde MutCirc) et D (ADN cible de type sauvage / sonde WTCirc) ; Puits 4 : produit d'amplification par THRCA du substrat D ; Puits 5 : marqueur de poids moléculaire 100bp NEB.

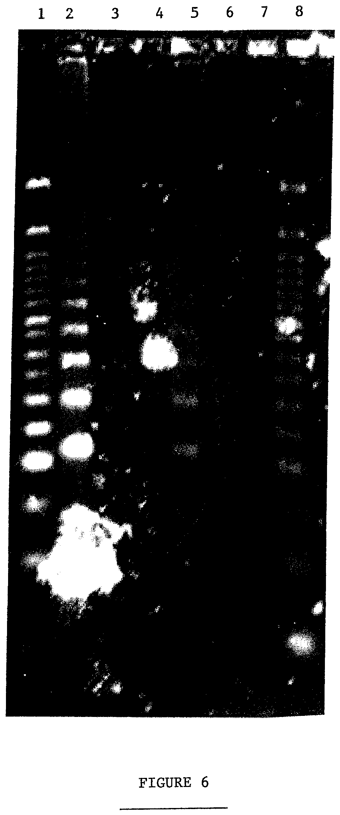

**Figure 6** : photographie de gel mettant en évidence l'importance du tampon au cours de l'amplification par THRCA en « une étape ». Puits 1 et 8 : marqueur de poids moléculaire 50bp NEB ; Puits 2 : produit d'amplification du substrat ADN cible de type sauvage / sonde WTCirc dans le tampon 4 modifié ; Puits 3 : produit d'amplification du substrat ADN cible de type sauvage / sonde WTCirc dans le tampon de la ligase + BSA ; Puits 4 : produit d'amplification du substrat ADN cible de type sauvage / sonde WTCirc dans le tampon de la polymérase + rATP ; Puits 5 : produit d'amplification du substrat ADN cible de type mutant / sonde WTCirc dans le tampon 4 modifié ; Puits 6 : produit d'amplification du substrat ADN cible de type mutant / sonde WTCirc dans le tampon de la ligase + BSA ; Puits 7 : produit d'amplification du substrat ADN cible de type mutant / sonde WTCirc dans le tampon de la polymérase + rATP.

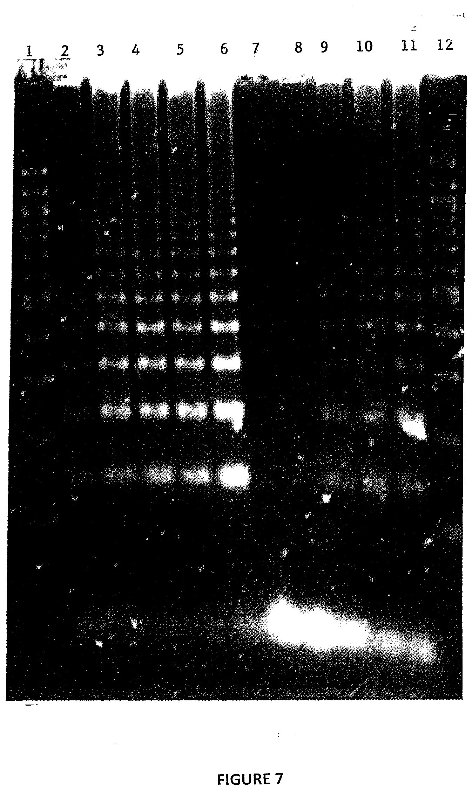

**Figure 7** : photographie de gel mettant en évidence la cinétique de la réaction d'amplification par THRCA en « une étape ». Puits 1 et 12 : marqueur de poids moléculaire 100bp NEB ; Puits 2 à 6 : produits d'amplification du substrat ADN cible de type sauvage / sonde WTCirc (témoin positif) obtenus respectivement après 15 min (puits 2), 25 min (puits 3), 35 min (puits 4), 45 min (puits 5) et 50 min (puits 6) de réaction ; Puits 7 à 11 : produit d'amplification du substrat ADN cible de type mutant / sonde WTCirc (témoin négatif) respectivement après 15 min (puits 7), 25 min (puits 8), 35 min (puits 9), 45 min (puits 10) et 50 min (puits 11) de réaction.

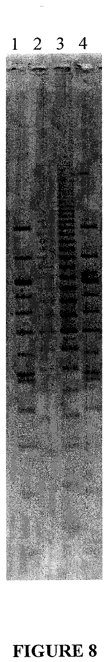

**Figure 8** : photographie de gel mettant en évidence le blocage de la polymérase au cours de la THRCA. Puits 1 et 4 : marqueur de poids moléculaire 100bp NEB ; Puits 2 : produit d'amplification par THRCA du substrat D (ADN cible de type sauvage / sonde WTCirc) avec le couple d'amorces WTFP (sans espaceur) / WTRP (sans fluorophore) ; Puits 3 : produit d'amplification par THRCA du substrat D avec le couple d'amorces WTFP (avec espaceur) /WTRP (sans fluorophore).

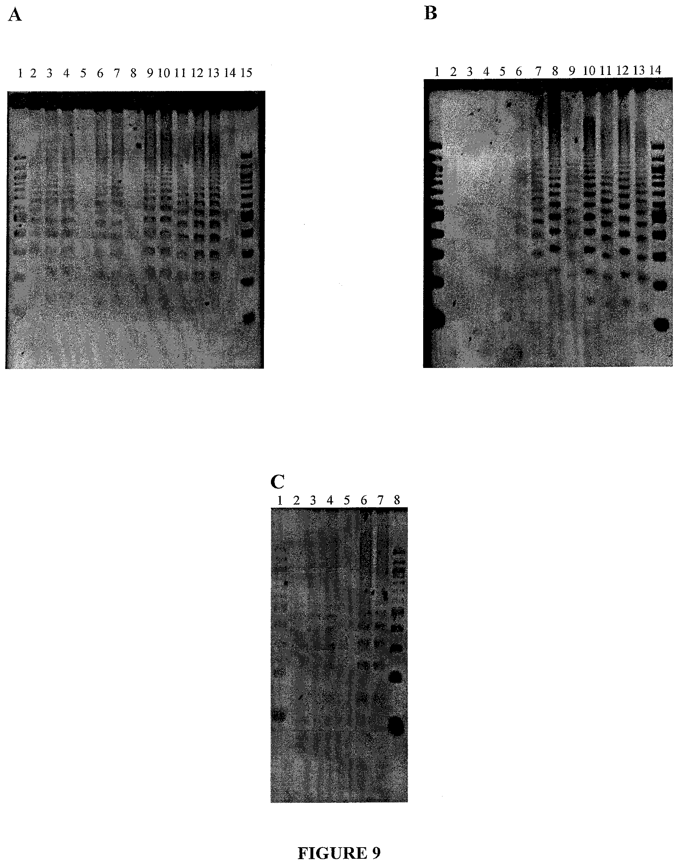

**Figure 9** : photographies de gel mettant en évidence l'optimisation du temps d'amplification pour la THRCA. **A** : Puits 1 et 15 : marqueur de poids moléculaire 100bp NEB ; Puits 2, 3 et 4 : produits d'amplification du substrat A (ADN cible de type mutant / sonde MutCirc ; réaction de ligation en présence de la ligase) obtenus respectivement après 30, 60 et 90 min de réaction THRCA ; Puits 5, 6 et 7 : produits d'amplification du substrat B (ADN cible de type mutant / sonde WTCirc ; réaction de ligation en présence de la ligase) obtenus respectivement après 30, 60 et 90 min de réaction THRCA ; Puits 8, 9 et 10 : produits d'amplification du substrat C (ADN cible de type sauvage / sonde MutCirc ; réaction de ligation en présence de la ligase) obtenus respectivement après 30, 60 et 90 min réaction THRCA ; Puits 11, 12 et 13 : produits d'amplification du substrat D (ADN cible de type sauvage / sonde WTCirc ; réaction de ligation en présence de la ligase) obtenus respectivement après 30, 60 et 90 min de réaction THRCA ; Puits 14 : vide. **B** Puits 1 et 14 : marqueur de poids moléculaire 100bp NEB ; Puits 2 : produit d'amplification du substrat A- (ADN cible de type mutant / sonde MutCirc ; réaction de ligation en l'absence de ligase) obtenu après 30 min de réaction THRCA ; Puits 3 : produit d'amplification du substrat B- (ADN cible de type mutant / sonde WTCirc ; réaction de ligation en l'absence de ligase) obtenu après 30 min de réaction THRCA ; Puits 4 : produit d'amplification du substrat C- (ADN cible de type sauvage / sonde MutCirc ; réaction de ligation en l'absence de ligase) obtenu après 30 min de réaction THRCA ; Puits 5 : produit d'amplification du substrat D- (ADN cible de type sauvage / sonde WTCirc ; réaction de ligation en l'absence de ligase) obtenu après 30 min de réaction THRCA ; Puits 6, 7, 8 et 9 : produits d'amplification des substrats A-, B-, C- et D-respectivement obtenus après 60 min de réaction THRCA ; Puits 10, 11, 12 et 13 : produits d'amplification des substrats A-, B-, C- et D- respectivement obtenus après 90 min de réaction THRCA. **C** : Puits 1 et 8 : marqueur de poids moléculaire 100bp NEB ; Puits 2, 3 et 4 : produits d'amplification du substrat E (sonde WTCirc ; réaction de ligation en présence de ligase) obtenus respectivement après 30, 60 et 90 min de réaction THRCA ; Puits 5, 6 et 7 : produits d'amplification du substrat F (sonde MutCirc ; réaction de ligation en présence de ligase) obtenus respectivement après 30, 60 et 90 min de réaction THRCA.

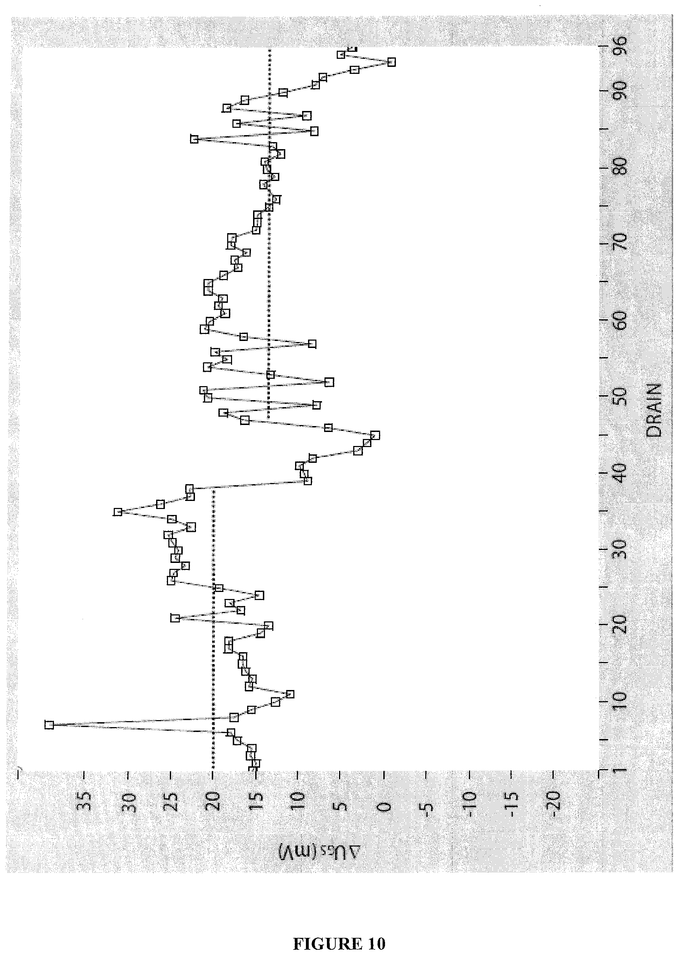

**Figure 10** **:** graphique représentant le résultat de la mesure électronique différentielle entre la sonde oligonucléotidique Ars3 (drains 1-38) et la sonde oligonucléotidique Ars5 (drains 47-96) après hybridation du produit d'amplification par THRCA du substrat D (ADN cible de type sauvage / sonde WTCirc) en utilisant les couples d'amorces suivants WTFP/WTRP
**Figure 11** : schéma représentant deux transistors à effet de champ d'une puce de détection comprenant une pluralité de tels transistors organisés suivant un réseau mono ou bidimensionnel de transistors.

### EXEMPLE 1 : MISE EN ŒUVRE DE LA THRCA

### 1-1. Matériels et Méthodes

### a) Séquences des oligonucléotides

Tous les oligonucléotides utilisés ont été synthétisés par la société Eurogentec.

### ADN cible de type sauvage (WT) :

5'-GCT ACT CGC TGA AAT TAA TAC GAC TCA CTA GGT GCC ACG G-3' (SEQ ID NO : 1). Cette séquence peut représenter par exemple un fragment d'un allèle de type sauvage.

### ADN cible de type mutant (Mut) :

5'-GCT ACT CGC TGA AAT TAA TAC GA**A** TCA CTA GGT GCC ACG G-3' (SEQ ID NO : 2). La séquence de l'ADN cible de type mutant diffère de la séquence de l'ADN cible de type sauvage en ce que la cytosine (C) en position 24 de la séquence de l'ADN cible de type sauvage a été substituée par une adénine (A). Cette séquence peut représenter par exemple un fragment d'un allèle de type mutant qui comprendrait un polymorphisme d'une seule paire de base (SNP).

### Sonde (ADN monocaténaire) circularisable de type sauvage (WTCirc) :

5'-[Phos]TCG TAT TAA TTT CAG CGA GTG GGA *TCG GCG CAC CTG CCG* *GAA* AGG CCG AAT TCA ACG GTT GTG GTC TCC CTA ACC TAG TGA G-3' (SEQ ID NO : 3).

### Sonde (ADN monocaténaire) circularisable de type mutant (MutCirc) :

5'-[Phos] TCG TAT TAA TTT CAG CGA GTT TCT GAC *TCG TCA TGT CTC AGC TCT AGT* *ACG* CTG ATC TTA GTG TCA GGA TAC GGT GTA GAC CTA GTG AT-3' (SEQ ID NO : 4).

Les sondes comportent un groupe phosphate (noté [Phos], PO₃²⁻) en position 5' afin de permettre leur circularisation.

### Amorce sens de type sauvage (Forward Primer WT, WTFP) :

5'-AAC GTC AGC CCT GCC GCC TG****TTC CGG CAG GTG CGC CGA-3' (SEQ ID NO : 5). **** correspond à l'espaceur de formule I :

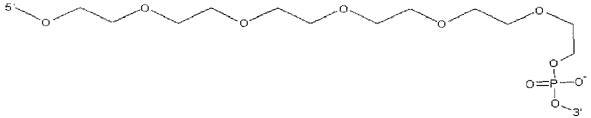

Le code barre de l'amorce est représenté par le fragment nucléotidique défini par les positions 1-20 incluses. Le fragment nucléotidique défini par les positions 21-38 incluses est apte à s'hybrider au fragment nucléotidique défini par les positions 25 à 42 incluses (en italique) de la sonde WTCirc.

### Amorce anti-sens de type sauvage (Reverse Primer WT, WTRP) :

5'-[Cy5/Cy3] CTA AGC TGT AGC CGG AGT GAA GGC CGA ATT CAA CGG TTG TGG-3' (SEQ ID NO : 6). L'amorce WTRP est marquée avec les fluorophores Cy5 ou Cy3 en position 5'. Le fragment nucléotidique défini par les positions 1 à 19 incluses correspond à une séquence code barre (appelée séquence de liaison). Le fragment nucléotidique défini par les positions 20 à 42 incluses (souligné) est identique au fragment nucléotidique défini par les positions 42 à 64 incluses (souligné) de la sonde WTCirc.

### Amorce sens de type mutant (Forward Primer mutant, MutFP) :

5'-GGC GGA GAG TCA GTT CGC GG**** GTA CTA GAG CTG AGA CAT GAC GAG TC-3' (SEQ ID NO : 7). **** correspond à l'espaceur de formule I définie ci-dessus. Le code barre de l'amorce est représenté par le fragment nucléotidique défini par les positions 1-20 incluses de l'amorce MutFP. Le fragment nucléotidique défini par les positions 21-47 incluses est apte à s'hybrider au fragment nucléotidique défini par les positions 27 à 50 incluses (en italique) de la sonde MutCirc.

### Amorce anti-sens de type mutant (Reverse Primer mutant, MutRP) :

5'-[Cy3] CTA AGC TGT AGC CGG AGT GAG CTG ATC TTA GTG TCA GGA TAC GG-3' (SEQ ID NO : 8). L'amorce MutRP est marquée avec le fluorophore Cy3 en position 5'. Le fragment nucléotidique défini par les positions 1 à 20 incluses correspond à une séquence de liaison. Le fragment nucléotidique défini par les positions 21-44 incluses (souligné) est identique au fragment nucléotidique défini par les positions 51 à 74 incluses (souligné) de la sonde MutCirc.

### b) Ligation

Les sondes nucléotidique sont mélangées aux ADN cibles afin d'être circularisées par action d'une ligase thermostable : 5U d'Ampligase (Epicentre Technologies) sont ajoutées à un mélange de 30 µL contenant 20mM Tris HCl (pH 8,3) ; 25mM KCl ; 10mM MgCl2 ; 0,5mM NAD ; 0,01 % Triton X-100 ; 300nM de sonde nucléotidique et 33nM d'ADN cible. Le mélange ainsi obtenu est incubé à 95°C pendant 5 min, puis incubé à 60°C pendant 15 min pour permettre la ligation. Les produits ainsi obtenus sont conservés à 4 °C.

### c) Amplification des sondes circularisées par THRCA.

Un aliquot des produits de ligation dilués 1000 fois est ajouté à un mélange de 25µL contenant 20mM Tris HCl (pH 8.8); 10mM KCl ; 10mM (NH4)2SO4 ; 2mM MgS04 ; 0,1% Triton X-100 ; 200µM dNTPs ; 1µM d'amorces sens et antisens ; 2U de Bst DNA polymérase (NEB). Le mélange ainsi obtenu est incubé à 62°C pendant 30 min. 2µL des produits d'amplification obtenus sont ensuite examinés en les chargeant sur un gel d'agarose 1,5 % pour électrophorèse.

### d) Amplification par THRCA en « une étape » (« One Step »).

60nM de sonde nucléotidique et 8nM d'ADN cible sont ajoutés à un mélange de 30µL contenant :
- le tampon du NEBuffer 4 (NEB, constitué de 20mM Tris acetate ; 50 mM potassium acétate ; 10mM magnesium acétate ; 1mM Dithiothréitol) ; 100µg/ml BSA ; 1mM rATP (dénommé « tampon 4 modifié »); ou
- le tampon de la ligase « Ampligase » (Epicentre Technologies) ; 100µg/ml BSA ; ou
- le tampon de la polymérase « Bst DNA polymérase » (NEB ; Remarque : ce tampon contient du Triton X-100) ; 1mM rATP ;
et
200µM dNTPs ; 1µM d'amorces sens et antisens ; 2U d'Ampligase (Epicentre Technologies), et 2U de Bst DNA polymérase (NEB).

Le mélange ainsi obtenu est incubé à 62°C pendant 35 min. 2µL des produits d'amplification obtenus sont ensuite examinés en les chargeant sur un gel d'agarose 1,5 % pour électrophorèse.

### 1-2. Résultats

### a) Mise en évidence du blocage de la polymérisation au cours de la THRCA

Une amplification par THRCA de la sonde WTCirc, circularisée et hybridée à l'ADN cible de type sauvage (appelé substrat D), à l'aide des amorces WTFP (avec ou sans espaceur) et WTRP, a été effectuée. Les résultats sont représentés à la Figure 3. Comme on peut le constater sur la photographie du gel, il existe une différence de migration entre les produits d'amplification obtenus en utilisant l'amorce WTFP avec espaceur (polymérisation bloquée) ou l'amorce WTFP sans espaceur (polymérisation non bloquée). En effet, lorsque l'ADN polymérase arrive au niveau de l'espaceur, elle rencontre les unités éthylène glycol formant l'espaceur et ne peut donc pas continuer la synthèse. Le blocage de l'ADN polymérase au cours de la synthèse (polymérisation) est donc effectif avec l'amorce WTFP comprenant un espaceur.

Par ailleurs, la présence du fluorophore (Cy3 ou Cy5) n'induit pas de différence de migration significative sur le gel. La différence de migration est donc uniquement due au blocage de la polymérisation.

Le substrat D (ADN cible de type sauvage / sonde WTCirc) a aussi été soumis à une amplification par THRCA en utilisant soit le couple d'amorces WTFP (avec espaceur)/WTRP (sans fluorophore) soit le couple d'amorces WTFP (sans espaceur)/WTRP(sans fluorophore). Les résultats sont représentés à la Figure 8, qui montre le blocage de la polymérase lorsqu'est utilisé le couple d'amorces WTFP (avec espaceur)/WTRP (sans fluorophore).

### b) Spécificité de la THRCA

Dans l'optique d'un test clinique (détection d'un polymorphisme génétique) il est essentiel de s'assurer de la spécificité de l'amplification obtenue par THRCA.

Pour cela, 3 ligations ont été effectuées :
A : ADN cible de type mutant / sonde MutCirc
B : ADN cible de type mutant / sonde WTCirc
C : ADN cible de type sauvage / sonde MutCirc

Les substrats A, B et C ont ensuite été soumis à une amplification par THRCA en utilisant respectivement les couples d'amorces suivants : MutFP/MutRP, WTFP/WTRP et MutFP/MutRP.

Les résultats sont représentés à la Figure 4. Conformément à ce qui était attendu, il n'y a pas amplification des produits ayant pour substrat B ou C, car les sondes ne s'hybrident pas entièrement à l'ADN cible. On peut donc en déduire qu'il y a bien une spécificité de l'amplification par THRCA.

### c) Amplification par THRCA avec un multiplexage

La compatibilité de la méthode THRCA avec un multiplexage (c'est-à-dire plusieurs réactions d'amplification dans le même milieu réactionnel) a été vérifiée avec deux réactions simultanées.

Pour cela, 2 ligations ont été effectuées :
A : ADN cible de type mutant / sonde MutCirc
D : ADN cible de type sauvage / sonde WTCirc

Les substrats A, D et A + D ont ensuite été soumis à une amplification par THRCA en utilisant respectivement les couples d'amorces suivants: MutFP/MutRP (pour le produit A), WTFP/WTRP sans espaceur (pour le produit D) et MutFP/MutRP + WTFP/WTRP (pour le produit A + D).

Les résultats sont représentés à la Figure 5. On constate que pour le puits 3 (produit de THRCA amplifié à partir des substrats A + D) il y a une amplification qui correspond à l'amplification de A et D à la fois (doublement des bandes).

Remarque : le décalage entre les bandes des puits 2 et 4 est dû au fait que les sondes WTCirc et MutCirc n'ont pas la même taille et par l'utilisation d'amorces non bloquantes (ne comprenant pas d'espaceur) dans le puits 4 pour accentuer le décalage entre les bandes.

Cette expérience montre aussi qu'il est possible de détecter un individu hétérozygote pour un gène donné en mettant en oeuvre la technique THRCA.

Cette expérience peut être généralisée à la détection de plusieurs mutations pourvu qu'on utilise des sondes et des amorces supplémentaires.

### d) Amplification par THRCA en « une étape »

Le procédé d'amplification par THRCA en « une étape » correspond à une méthode d'amplification au cours de laquelle la ligation de la sonde hybridée à l'ADN cible et la polymérisation s'effectuent dans le même milieu réactionnel.

### Mise au point du tampon

Le pH des tampons « natifs » de la ligase et de la polymérase étant différents, il était nécessaire, dans un premier temps, de trouver un tampon unique dans lequel la ligase et la polymérase soient fonctionnelles.

L'amplification par THRCA en « une étape » du substrat ADN cible de type sauvage / sonde WTCirc ou du substrat ADN cible de type mutant / sonde WTCirc (témoin négatif) a été testée avec 3 tampons différents :
i) le tampon NEBuffer 4 (NEB) auquel sont ajoutés des rATPs (nécessaires pour la ligase) et de la BSA (nécessaire pour la polymérase) (dénommé « tampon 4 modifié ») ;
ii) le tampon de la ligase « Ampligase » commercialisé par Epicentre Technologies, auquel est ajoutée de la BSA (nécessaire pour la polymérase) ;
iii) le tampon de la polymérase « Bst DNA polymérase » commercialisé par NEB, auquel sont ajoutés des rATPs (nécessaires pour la ligase).

Les résultats sont représentés à la Figure 6. Comme le montre la photographie du gel, seule une amplification, dans le tampon 4 modifié, du substrat ADN cible de type sauvage / sonde WTCirc a été obtenue.

Toutefois on remarque également que l'on perd la spécificité de la réaction comme le montre l'amplification du substrat ADN cible de type mutant / sonde WTCirc (témoin négatif). On note cependant que l'amplification du témoin négatif est plus faible que celle de l'amplification avec le substrat positif (ADN cible de type sauvage / sonde WTCirc). Cela suggère qu'il existe une fenêtre temporelle sur laquelle on retrouve la spécificité.

### Cinétique du procédé d'amplification par THRCA en « une étape »

Il ressort des résultats décrits au paragraphe précédent que la cinétique est un facteur important pour garantir la spécificité de l'amplification dans ces conditions expérimentales.

Il a donc été effectuée une cinétique de la réaction de THRCA à partir des substrats ADN cible de type sauvage / sonde WTCirc (témoin positif) ou ADN cible de type mutant / sonde WTCirc (témoin négatif), dans le tampon 4 modifié, en prélevant à t=15min, t=25 min, t= 35min, t=45 min et t=50min 2µL du mélange réactionnel.

Les résultats sont représentés à la Figure 7. Il ressort de la photographie du gel, que l'amplification des produits (-) a lieu entre 25 et 35 minutes après le début de l'incubation tandis que celle des produits (+) a lieu à partir de 15 minutes d'incubation.

On en déduit donc que dans ces conditions expérimentales, l'amplification par THRCA en « une étape » dans le tampon 4 modifié pendant environ 30 minutes permet d'obtenir une amplification spécifique de la sonde circularisée hybridée à l'ADN cible ; ce qui est compatible avec une application clinique.

### e) Détermination du temps d'amplification optimal lors de l'amplification THRCA

10 ligations, en présence ou en l'absence de ligase, ont été effectuées conformément au protocole indiqué ci-dessus (paragraphe 1.1-b) :
- A : ADN cible de type mutant / sonde MutCirc (présence de la ligase dans le mélange réactionnel de ligation)
- B : ADN cible de type mutant / sonde WTCirc (présence de la ligase dans le mélange réactionnel de ligation)
- C : ADN cible de type sauvage / sonde MutCirc (présence de la ligase dans le mélange réactionnel de ligation)
- D : ADN cible de type sauvage / sonde WTCirc (présence de la ligase dans le mélange réactionnel de ligation)
- A- : ADN cible de type mutant / sonde MutCirc, mais la ligase n'a pas été ajoutée au mélange réactionnel de ligation
- B- : ADN cible de type mutant / sonde WTCirc, mais la ligase n'a pas été ajoutée au mélange réactionnel de ligation
- C- : ADN cible de type sauvage / sonde MutCirc, mais la ligase n'a pas été ajoutée au mélange réactionnel de ligation
- D- : ADN cible de type sauvage / sonde WTCirc, mais la ligase n'a pas été ajoutée au mélange réactionnel de ligation
- E (témoin) : sonde WTCirc (présence de la ligase mélange réactionnel de ligation)
- F (témoin) : sonde MutCirc (présence de la ligase mélange réactionnel de ligation).

Les substrats A, B, C, D, A-, B-, C-, D-, E et F ont ensuite été soumis à une amplification par THRCA, pendant 30, 60 ou 90 minutes, en utilisant le couple d'amorces MutFP/MutRP pour les substrats A, C, A-, C- et F, et le couple d'amorces WTFP/WTRP pour les substrats B, D, B-, D- et E.

Les résultats sont représentés à la Figure 9.

Figure 9A : Conformément à ce qui était attendu, il y a une amplification des substrats A et D à 30, 60 et 90 min et il n'y a pas d'amplification des substrats B et C à 30 min. Contrairement à ce qui était attendu, il y a une amplification des substrats B et C à 60 et 90 min.

Figure 9B : Conformément à ce qui était attendu, il n'y a pas d'amplification des substrats A-, B-, C- et D- à 30 min (car la ligation n'a pas pu s'effectuer en l'absence de ligase). Contrairement à ce qui était attendu, il y a une amplification des substrats A-, B-, C- et D- à 60 et 90 min.

Figure 9C : les sondes linéaires ne sont pas amplifiées à 30 min mais le sont à 60 et 90 min.

Ces résultats montrent que le temps d'amplification optimal doit être déterminé par l'homme du métier afin d'éviter d'obtenir de faux résultats positifs.

Dans le cas présent, un temps d'amplification de 30 min est optimal. Au-delà de ce temps, les sondes linéaires sont aussi amplifiées lors de l'amplification par THRCA.

### EXEMPLE 2 : DETECTION ELECTRONIQUE DE PRODUITS D'AMPLIFICATION OBTENUS PAR THRCA

Le substrat D défini ci-dessus (ADN cible de type sauvage / sonde WTCirc) a été soumis à une amplification par THRCA, selon le protocole de détection en deux étapes (ligation puis amplification) en utilisant le couple d'amorces WTFP/WTRP.

Les produits d'amplifications par THRCA obtenus ont ensuite été détectés à l'aide d'une puce de détection électronique comprenant un réseau de transistors à effet de champ telle que décrite dans la Demande Internationale WO 2004/057027 (voir aussi Figure 11), en suivant sensiblement le même protocole que celui décrit à l'Exemple 1 de cette Demande Internationale.

### 2.1 Matériel

- NaOH : 60 ml de NaOH 16N, 420 ml d'éthanol et 220 ml d'H₂O
- H₂SO₄ 1M
- PLL : solution de poly-L-lysine, P8920 (Sigma), 0.01% w/v dans un tampon PBS 0.1x
- sonde oligonucléotidique Ars3 : 5'-CCG CGA ACT GAC TCT CCG CC-3' (SEQ ID NO: 9), complémentaire de la séquence code barre de l'amorce MutFP (décrite à l'Exemple 1)
- sonde oligonucléotidique Ars5 : 5'-CAG GCG GCA GGG CTG ACG TT-3' (SEQ ID NO: 10), complémentaire de la séquence code barre de l'amorce WTFP (décrite à l'Exemple 1)

### 2.2 Méthode

### Traitement global de la surface en silicium (SiO₂) des transistors à effet de champ

Incubation pendant 1 min dans de l'acide sulfurique (H₂SO₄), puis rinçage sous un courant d'eau déionisée, et séchage à l'air comprimé. Ce cycle d'incubation / rinçage / séchage est répété une fois. Incubation 4 min dans la solution de NaOH, puis rinçage à l'eau et séchage.

### Mesure électronique après traitement NaOH

Tampon de mesure : KCl à la concentration de 0,01 mM. Cette mesure est suivie d'un rinçage à l'eau et d'un séchage.

### Traitement global avec la poly-L-lysine

Incubation avec la poly-L-lysine pendant 2h, puis rinçage à l'eau et séchage.

### Mesure électronique "PL1"

Tampon de mesure : KCl 0,01 mM. Cette mesure est suivie d'un rinçage à l'eau et d'un séchage.

### Mesure électronique "PL2"

Tampon de mesure : KCl 0,01 mM. Cette mesure est suivie d'un rinçage à l'eau et d'un séchage. Cette deuxième mesure a pour but de vérifier la stabilité de la mesure à ce stade.

### Dépôt des sondes oligonucléotidiques

0,2 µl d'une solution contenant la sonde oligonucléotidique Ars3 a été déposé à l'aide d'une micropipette sur la partie gauche du réseau de transistors à effet de champ (drains 1-38). 0,2 µl d'une solution contenant la sonde oligonucléotidique Ars5 a été déposé sur la partie droite du réseau de transistors à effet de champ (drains 47-96). Dans les deux cas, les solutions contiennent 1 µM d'oligonucléotide dans un tampon KCl 20 mM. Incubation pendant 15 minutes, en atmosphère humide, suivi d'un rinçage à l'eau et d'un séchage.

### Mesure électronique "Sonde"

Mesure effectuée avec un tampon de mesure constitué de KCl 0,01 mM, puis pompage de l'électrolyte et remplacement par 1 ml de KCl à 50 mM, sans séchage.

### Hybridation du produit d'amplification THRCA

Pompage du tampon jusqu'à ce qu'il ne reste que 30 µl. Injection de 0.7 µl de produit d'amplification THRCA (1,4 µM) (la dilution finale en produit THRCA est alors de l'ordre de 30 nM). Agitation par pompage. Le dépôt et l'agitation du produit d'amplification THRCA sont répétés 2 fois. Après l'agitation, incubation pendant 5 min suivie d'un rinçage avec du KCl 50 mM. Ce rinçage est réitéré 3 fois. Ensuite, rinçage par pompage de l'électrolyte et ajout de 1 ml de KCl 0,01 mM, et agitation, suivie d'un nouveau pompage. Ce cycle est réitéré 3 fois.

### Mesure électronique après hybridation

Plonger l'électrode de mesure dans un tampon de KCl 0,01 mM et effectuer la mesure électronique.

### 2.3 Résultat

Le résultat de la mesure électronique est représenté à la Figure 10. La figure montre les différences DeltaUGS entre la mesure électronique "après hybridation" et la mesure électronique "Sonde" Le décalage moyen pour la « région Ars5 » (drains 47-96) est plus négatif de 7 mV que celui de la « région Ars3 » (drains 1-38). Cette différence de potentiel est la signature d'une hybridation entre la sonde oligonucléotidique Ars5 et les produits THRCA portant le code barre de l'amorce WTFP.

## Revendications

1. Procédé de détection d'un ADN monocaténaire circularisé par amplification ramifiée isotherme par cercle roulant (HRCA) dudit ADN monocaténaire circularisé en présence d'une amorce sens apte à s'hybrider audit ADN monocaténaire circularisé et aux brins négatifs générés lors de la HRCA et d'une amorce anti-sens apte à s'hybrider aux brins positifs générés lors de la HRCA, ladite HRCA générant des ADN bicaténaires périodiques, lequel procédé étant **caractérisé en ce que** :
• ladite amorce sens est constituée de, ou comprend, de son extrémité 5' vers son extrémité 3', la séquence suivante : 5'-(F1)ₙ₁-T1-E1-A1-3', dans laquelle :
∘ F1 représente un groupement terminal choisi parmi un marqueur et un agent de couplage ;
∘ T1 représente une séquence nucléotidique code-barre constituée de 6 à 30 nucléotides ;
∘ E1 représente un espaceur qui bloque la polymérisation du brin complémentaire à ladite séquence nucléotidique T1 par une ADN polymérase dépourvue d'activité exonucléasique et possédant une activité de déplacement de brin ;
∘ A1 représente une séquence nucléotidique, constituée de 10 à 40 nucléotides, apte à s'hybrider audit ADN monocaténaire circularisé et auxdits brins négatifs ; et
∘ n1 est un nombre entier égal à 0 ou 1 ;
et/ou
• ladite amorce anti-sens est constituée de, ou comprend, de son extrémité 5' vers son extrémité 3', la séquence suivante : 5'-(F2)ₙ₂-T2-E2-A2-3', dans laquelle :
∘ F2 représente un groupement terminal, choisi parmi un marqueur et un agent de couplage, qui peut être identique au ou différent du groupement terminal F1 ;
∘ T2 représente une séquence nucléotidique code-barre, constituée de 6 à 30 nucléotides, qui peut être identique à ou différente de la séquence nucléotidique T1 ;
∘ E2 représente un espaceur qui bloque la polymérisation du brin complémentaire à ladite séquence nucléotidique T2 par une ADN polymérase dépourvue d'activité exonucléasique et possédant une activité de déplacement de brin, et qui peut être identique à ou différent de l'espaceur E1 ;
∘ A2 représente une séquence nucléotidique, constituée de 10 à 40 nucléotides, apte à s'hybrider auxdits brins positifs ; et
∘ n2 est un nombre entier égal à 0 ou 1 ;
ledit ADN monocaténaire circularisé étant détecté par hybridation desdites séquences codes-barres T1 et/ou T2 présentes aux extrémités desdits acides nucléiques périodiques bicaténaires à une sonde nucléotidique complémentaire auxdites séquences codes-barres T1 et/ou T2, où ladite sonde nucléotidique est présente sur un support solide.

2. Procédé selon la revendication 1, **caractérisé en ce que** l'espaceur E1 et/ou E2 est choisi dans le groupe constitué par un site abasique et un groupement alkyle, alcényle ou alcynyle linéaire ou ramifié, éventuellement substitué.

3. Procédé selon la revendication 2, **caractérisé en ce que** l'espaceur E1 et/ou E2 est constitué de ou comprend un polyéthylène glycol constitué d'un enchaînement de 1 à 100, de préférence de 4 à 8 unités éthylène glycol, et d'un groupement PO₃ à l'une de ses extrémités.

4. Procédé selon la revendication 3, **caractérisé en ce que** l'espaceur E1 et/ou E2 est de formule I : 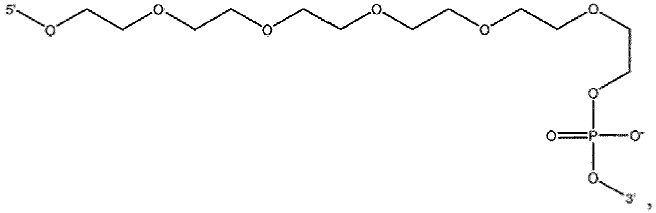 l'extrémité 5' dudit espaceur étant liée à l'extrémité 3' de ladite séquence T1 et/ou de ladite séquence T2 et l'extrémité 3' dudit espaceur étant liée à l'extrémité 5' de ladite séquence A1 et/ou de ladite séquence A2.

5. Procédé selon l'une quelconque des revendications 1 à 4, **caractérisé en ce que** ledit marqueur est choisi dans le groupe constitué par un agent luminescent, un radioisotope, une enzyme, la biotine, l'acrylamide, un thiol et un phosphorothioate.

6. Procédé de détection d'un polymorphisme génétique d'une seule ou de plusieurs paire(s) de bases, comprenant les étapes suivantes :
i) mise en contact d'un acide nucléique cible susceptible de contenir une ou plusieurs bases polymorphes à détecter avec un ADN monocaténaire circularisable (sonde) ;
ii) hybridation dudit ADN monocaténaire à l'acide nucléique cible ;
iii) obtention, si l'acide nucléique cible contient la ou lesdites bases polymorphes, d'un ADN monocaténaire hybridé et circularisé par ligation dudit ADN monocaténaire hybridé à l'acide nucléique cible en présence d'une ADN ligase ;
iv) obtention d'ADN bicaténaires périodiques par amplification ramifiée isotherme par cercle roulant dudit ADN monocaténaire hybridé et circularisé, en présence d'au moins l'une des deux amorces de séquences 5'-(F1)ₙ₁-T1-E1-A1-3' et 5'-(F2)ₙ₂-T2-E2-A2-3' telles que définies aux revendications 1 à 5 ;
v) détection des séquences codes-barres T1 et/ou T2 présentes aux extrémités des acides nucléiques périodiques bicaténaires obtenus à l'étape iv) par hybridation à une sonde nucléotidique complémentaire auxdites séquences codes-barres T1 et/ou T2, où ladite sonde nucléotidique est présente sur un support solide ; la détection des séquences codes-barres T1 et/ou T2 présentes aux extrémités desdits ADN périodiques bicaténaires indiquant que ledit acide nucléique cible contient la ou lesdites bases polymorphes.

7. Procédé selon la revendication 6, **caractérisé en ce que** les étapes i) à iv) sont réalisées par mélange dudit ADN monocaténaire circularisable, dudit acide nucléique cible, desdites amorces, de désoxynucléotides, d'une ADN ligase, de rATP, d'une ADN polymérase dépourvue d'activité exonucléasique et possédant une activité de déplacement de brin, d'un stabilisateur de ladite polymérase et d'un tampon approprié dans lequel ladite ADN ligase circularise l'ADN monocaténaire hybridé à l'ADN cible si l'hybridation ne contient aucun mésappariement et ladite ADN polymérase catalyse la polymérisation des brins d'ADN.

8. Procédé selon la revendication 7, **caractérisé en ce que** ledit tampon comprend de l'acétate de potassium, du tris-acétate, de l'acétate de magnésium et du dithiothréitol ou en est constitué.

9. Procédé selon l'une quelconque des revendications 1 à 8, **caractérisé en ce que** lesdites séquences codes-barres T1 et/ou T2 sont détectées par une technique de détection par hybridation dans laquelle le signal de détection de l'hybridation entre la séquence code-barre et une séquence nucléotidique (sonde) complémentaire à ladite séquence code-barre augmente avec le nombre de nucléotides de la molécule d'acide nucléique comprenant ladite séquence code-barre.

10. Procédé selon la revendication 9, **caractérisé en ce que** lesdites séquences codes-barres T1 et/ou T2 sont détectées par une méthode de détection électronique.

11. Utilisation d'au moins une paire d'amorces sens et anti-sens pour la détection d'un ADN monocaténaire circularisé par amplification ramifiée isotherme par cercle roulant (HRCA) ;
dans laquelle l'amorce sens est apte à s'hybrider audit ADN monocaténaire circularisé et aux brins négatifs générés lors de la HRCA, et l'amorce anti-sens est apte à s'hybrider aux brins positifs générés lors de la HRCA ;
et dans laquelle ladite amorce sens est constituée de, ou comprend, de son extrémité 5' vers son extrémité 3', la séquence suivante : 5'-(F1)ₙ₁-T1-E1-A1-3', telle que définie à l'une quelconque des revendications 1 à 5 et/ou ladite amorce anti-sens est constituée de, ou comprend, de son extrémité 5' vers son extrémité 3', la séquence suivante : 5'-(F2)ₙ₂-T2-E2-A2-3', telle que définie à l'une quelconque des revendications 1 à 5 ;
et d'un support solide comprenant des séquences nucléotidiques qui sont complémentaires à la séquence code-barre.

12. Kit ou coffret de détection d'un polymorphisme génétique d'une seule ou de plusieurs paire(s) de bases, **caractérisé en ce qu'**il comprend au moins une paire d'amorces sens et anti-sens dans laquelle ladite amorce sens est constituée de, ou comprend, de son extrémité 5' vers son extrémité 3', la séquence suivante : 5'-(F1)ₙ₁-T1-E1-A1-3', telle que définie à l'une quelconque des revendications 1 à 5 et/ou ladite amorce anti-sens est constituée de, ou comprend, de son extrémité 5' vers son extrémité 3', la séquence suivante : 5'-(F2)ₙ₂-T2-E2-A2-3', telle que définie à l'une quelconque des revendications 1 à 5, un ADN monocaténaire circularisable, une ADN ligase, une ADN polymérase dépourvue d'activité exonucléasique et possédant une activité de déplacement de brin, des désoxynucléotides, un tampon approprié et une puce à ADN ; où ladite puce à ADN porte des séquences nucléotidiques qui sont complémentaires à la séquence code-barre.

## Patentansprüche

1. Verfahren zum Nachweis einer zirkularisierten einzelsträngigen DNA durch isotherme hyperverzweigte Rolling-Circle-Amplifikation (HRCA) der zirkularisierten einzelsträngigen DNA in Gegenwart eines Sense-Primers, der an die zirkularisierte einzelsträngige DNA und an die während der HCRA erzeugten negativen Stränge hybridisieren kann, und eines Antisense-Primers, der an die während der HRCA erzeugten positiven Stränge hybridisieren kann, wobei die HRCA periodische doppelsträngige DNAs erzeugt, wobei das Verfahren **dadurch gekennzeichnet ist, dass**:
• der Sense-Primer von seinem 5'-Ende zu seinem 3'-Ende aus der folgenden Sequenz besteht oder diese umfasst: 5'-(F1)ₙ₁-T1-(E1)-A1-3', worin:
∘ F1 eine endständige Gruppe darstellt, die aus einem Marker und einem Kupplungsmittel ausgewählt ist;
∘ T1 eine aus 6 bis 30 Nukleotiden bestehende Barcode-Nukleotidsequenz darstellt;
∘ E1 einen Spacer darstellt, der die Polymerisation des zu der Nukleotidsequenz T1 komplementären Stranges durch eine DNA-Polymerase, die keine Exonukleaseaktivität besitzt und eine Strangverdrängungsaktivität besitzt, blockiert;
∘ A1 eine aus 10 bis 40 Nukleotiden bestehende Nukleotidsequenz darstellt, die an die zirkularisierte einzelsträngige DNA und an die negativen Stränge hybridisieren kann, und
∘ n1 eine ganze Zahl gleich 0 oder 1 ist;
und/oder
• der Antisense-Primer von seinem 5'-Ende zu seinem 3'-Ende aus der folgenden Sequenz besteht oder diese umfasst: 5'-(F2)ₙ₂-T2-(E2)-A2-3', worin:
∘ F2 eine aus einem Marker und einem Kupplungsmittel ausgewählte endständige Gruppe darstellt, die mit der endständigen Gruppe F1 identisch oder von dieser verschieden sein kann;
∘ T2 eine aus 6 bis 30 Nukleotiden bestehende Barcode-Nukleotidsequenz darstellt, die mit der Nukleotidsequenz T1 identisch oder von dieser verschieden sein kann;
∘ E2 einen Spacer darstellt, der die Polymerisation des zu der Nukleotidsequenz T2 komplementären Strangs durch eine DNA-Polymerase, die keine Exonukleaseaktivität besitzt und eine Strangverdrängungsaktivität besitzt, blockiert und der mit dem Spacer E1 identisch oder von diesem verschieden sein kann;
∘ A2 eine aus 10 bis 40 Nukleotiden bestehende Nukleotidsequenz darstellt, die an die positiven Stränge hybridisieren kann, und
∘ n2 eine ganze Zahl gleich 0 oder 1 ist;
wobei die zirkularisierte einzelsträngige DNA durch Hybridisierung der Barcode-Sequenzen T1 und/oder T2, die an den Enden der periodischen doppelsträngigen Nukleinsäuren vorhanden sind, an eine zu den Barcode-Sequenzen T1 und/oder T2 komplementäre Nukleotidsonde nachgewiesen wird, wobei die Nukleotidsonde auf einem festen Träger vorliegt.

2. Verfahren nach Anspruch 1, **dadurch gekennzeichnet, dass** der Spacer E1 und/oder E2 aus der Gruppe ausgewählt ist, bestehend aus einer abasischen Stelle und einer geraden oder verzweigten, gegebenenfalls substituierten, Alkyl-, Alkenyl oder Alkinylgruppe.

3. Verfahren nach Anspruch 2, **dadurch gekennzeichnet, dass** der Spacer E1 und/oder E2 aus einem Polyethylenglykol, das aus einer Aneinanderreihung von 1 bis 100, vorzugsweise 4 bis 8, Ethylenglykoleinheiten besteht, und einer Gruppe PO₃ an einem seiner Enden besteht oder diese umfasst.

4. Verfahren nach Anspruch 3, **dadurch gekennzeichnet, dass** der Spacer E1 und/oder E2 die folgende Formel I hat: 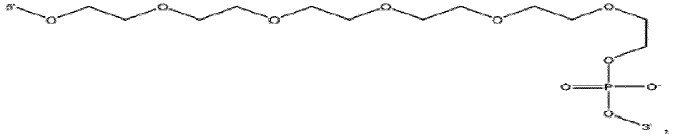 wobei das 5'-Ende des Spacers an das 3'-Ende der Sequenz T1 und/oder der Sequenz T2 gebunden ist und das 3'-Ende des Spacers an das 5'-Ende der Sequenz A1 und/oder der Sequenz A2 gebunden ist.

5. Verfahren nach einem der Ansprüche 1 bis 4, **dadurch gekennzeichnet, dass** der Marker aus der Gruppe ausgewählt ist, bestehend aus einem lumineszierenden Mittel, einem Radioisotop, einem Enzym, Biotin, Acrylamid, einem Thiol und einem Phosphorthioat.

6. Verfahren zum Nachweis eines genetischen Polymorphismus eines einzigen Basenpaars oder mehrerer Basenpaare, das die folgenden Schritte umfasst:
i) Inkontaktbringen einer Zielnukleinsäure, die eine oder mehrere nachzuweisende polymorphe Basen enthalten kann, mit einer zirkularisierbaren einzelsträngigen DNA (Sonde);
ii) Hybridisieren der einzelsträngigen DNA an die Zielnukleinsäure;
iii) wenn die Zielnukleinsäure die polymorphe Base oder die polymorphen Basen enthält, Erhalten einer hybridisierten und zirkularisierten einzelsträngigen DNA durch Ligation der hybridisierten einzelsträngigen DNA an die Zielnukleinsäure in Gegenwart einer DNA-Ligase;
iv) Erhalten periodischer doppelsträngiger DNA durch isotherme hyperverzweigte Rolling-Circle-Amplifikation der hybridisierten und zirkularisierten einzelsträngigen DNA in Gegenwart mindestens eines der zwei Primer mit den Sequenzen 5'-(F1)ₙ₁-T1-(E1)-A1-3' und 5'-(F2)ₙ₂-T2-(E2)-A2-3' nach den Ansprüchen 1 bis 5;
v) Nachweis der Barcode-Sequenzen T1 und/oder T2, die an den Enden der in Schritt iv) erhaltenen periodischen doppelsträngigen Nukleinsäuren vorhanden sind, durch Hybridisierung an eine zu den Barcode-Sequenzen T1 und/oder T2 komplementäre Nukleotidsonde, wobei die Nukleotidsonde auf einem festen Träger vorliegt; wobei der Nachweis der an den Enden der periodischen doppelsträngigen DNA vorhandenen Barcode-Sequenzen T1 und/oder T2 darauf hinweist, dass die Zielnukleinsäure die polymorphe Base bzw. die polymorphen Basen enthält.

7. Verfahren nach Anspruch 6, **dadurch gekennzeichnet, dass** die Schritte i) bis iv) durchgeführt werden durch Mischen der zirkularisierbaren einzelsträngigen DNA, der Zielnukleinsäure, der Primer, von Desoxynukleotiden, einer DNA-Ligase, rATP, einer DNA-Polymerase, die keine Exonukleaseaktivität besitzt und Strangverdrängungsaktivität besitzt, eines Stabilisators für die Polymerase und eines geeigneten Puffers, in dem die DNA-Ligase die an die Ziel-DNA hybridisierte einzelsträngige DNA zirkularisiert, wenn die Hybridisierung keine Fehlpaarungen enthält, und die DNA-Polymerase die Polymerisation der DNA-Stränge katalysiert.

8. Verfahren nach Anspruch 7, **dadurch gekennzeichnet, dass** der Puffer Kaliumacetat, Tris-Acetat, Magnesiumacetat und Dithiothreitol umfasst oder daraus besteht.

9. Verfahren nach einem der Ansprüche 1 bis 8, **dadurch gekennzeichnet, dass** die Barcode-Sequenzen T1 und/oder T2 durch eine Nachweistechnik mittels Hybridisierung nachgewiesen werden, wobei das Nachweissignal der Hybridisierung zwischen der Barcode-Sequenz und einer zu der Barcode-Sequenz komplementären Nukleotidsequenz (Sonde) sich mit der Anzahl der Nukleotide des Nukleinsäuremoleküls, das die Barcode-Sequenz umfasst, erhöht.

10. Verfahren nach Anspruch 9, **dadurch gekennzeichnet, dass** die Barcode-Sequenzen T1 und/oder T2 durch ein elektronisches Nachweisverfahren nachgewiesen werden.

11. Verwendung mindestens eines Paars von Sense- und Antisense-Primern zum Nachweis einer zirkularisierten einzelsträngigen DNA mittels isothermer hyperverzweigter Rolling-Circle-Amplifikation (HRCA), wobei der Sense-Primer an die zirkularisierte einzelsträngige DNA und an die während der HCRA erzeugten negativen Stränge hybridisieren kann und der Antisense-Primer an die während der HRCA erzeugten positiven Stränge hybridisieren kann;
und wobei der Sense-Primer von seinem 5'-Ende zu seinem 3'-Ende aus der folgenden Sequenz besteht oder diese umfasst: 5'-(F1)ₙ₁-T1-(E1)-A1-3', nach einem der Ansprüche 1 bis 5, und/oder der Antisense-Primer von seinem 5'-Ende zu seinem 3'-Ende aus der folgenden Sequenz besteht oder diese umfasst: 5'-(F2)ₙ₂-T2-(E2)-A2-3', nach einem der Ansprüche 1 bis 5,
und eines festen Trägers, der Nukleotidsequenzen umfasst, die zu der Barcode-Sequenz komplementär sind.

12. Kit oder Satz zum Nachweis eines genetischen Polymorphismus eines einzelnen Basenpaars oder mehrerer Basenpaare, **dadurch gekennzeichnet, dass** es/er mindestens ein Paar Sense- und Antisense-Primer, wobei der Sense-Primer von seinem 5'-Ende zu seinem 3'-Ende aus der folgenden Sequenz besteht oder diese umfasst: 5'-(F1)ₙ₁-T1-E1-A1-3', nach einem der Ansprüche 1 bis 5, und/oder der Antisense-Primer von seinem 5'-Ende zu seinem 3'-Ende aus der folgenden Sequenz besteht oder diese umfasst: 5'-(F2)ₙ₂-T2-E2-A2-3', nach einem der Ansprüche 1 bis 5, eine zirkularisierbare einzelsträngige DNA, eine DNA-Ligase, eine DNA-Polymerase, die keine Exonukleaseaktivität besitzt und eine Strangverdrängungsaktivität besitzt, Desoxynukleotide, einen geeigneten Puffer und einen DNA-Chip umfasst, wobei der DNA-Chip Nukleotidsequenzen trägt, die zu der Barcode-Sequenz komplementär sind.

## Claims

1. A method for detecting a circularized single-stranded DNA by hyperbranched rolling circle amplification (HRCA) of said circularized single-stranded DNA in the presence of a forward primer that is capable of hybridizing with said circularized single-stranded DNA and with negative strands generated during the HRCA, and a reverse primer that is capable of hybridizing with positive strands generated during the HRCA, said HRCA generating periodic double-stranded DNAs, said method being **characterized in that**:
• said forward primer is constituted by or comprises the following sequence, from its 5' end to its 3' end: 5'-(F1)ₙ₁-T1-E1-A1-3', in which:
∘ F1 represents a terminal group selected from a tag and a coupling agent;
∘ T1 represents a barcode nucleotide sequence constituted by 6 to 30 nucleotides;
∘ E1 represents a spacer that blocks polymerization of the strand complementary to said nucleotide sequence T1 by a DNA polymerase deprived of exonuclease activity and having a strand displacement activity;
∘ A1 represents a nucleotide sequence constituted by 10 to 40 nucleotides that is capable of hybridizing with said circularized single-stranded DNA and with said negative strands; and
∘ n1 is a whole number equal to 0 or 1;
and/or
• said reverse primer is constituted by or comprises the following sequence, from its 5' end to its 3' end: 5'-(F2)ₙ₂-T2-E2-A2-3', in which:
∘ F2 represents a terminal group selected from a tag and a coupling agent, which may be identical to or different from the terminal group F1;
∘ T2 represents a barcode nucleotide sequence constituted by 6 to 30 nucleotides, which may be identical to or different from the nucleotide sequence T1;
∘ E2 represents a spacer that blocks polymerization of the strand complementary to said nucleotide sequence T2 by a DNA polymerase deprived of exonuclease activity and having a strand displacement activity, and which may be identical to or different from the spacer E1;
∘ A2 represents a nucleotide sequence constituted by 10 to 40 nucleotides that is capable of hybridizing with said positive strands; and
∘ n2 is a whole number equal to 0 or 1;
said circularized single-stranded DNA being detected by hybridizing said barcode sequences T1 and/or T2 present at the ends of said double-stranded periodic nucleic acids with a nucleotide probe complementary to said barcode sequences T1 and/or T2, wherein said nucleotide probe is present on a solid support.

2. The method as claimed in claim 1, **characterized in that** the spacer E1 and/or E2 is selected from the group constituted by an abasic site and a linear or branched, optionally substituted alkyl, alkenyl or alkynyl group.

3. The method as claimed in claim 2, **characterized in that** the spacer E1 and/or E2 is constituted by or comprises a polyethylene glycol, constituted by a concatenation of 1 to 100, preferably 4 to 8 ethylene glycol units, and by a PO₃ group at one of its ends.

4. The method as claimed in claim 3, **characterized in that** the spacer E1 and/or E2 has formula I: 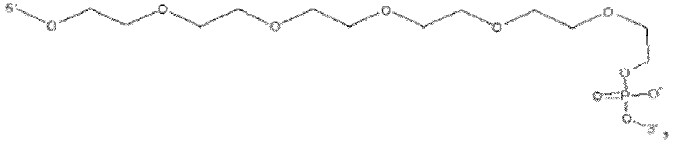 the 5' end of said spacer being bound to the 3' end of said sequence T1 and/or said sequence T2 and the 3' end of said spacer being bound to the 5' end of said sequence A1 and/or said sequence A2.

5. The method as claimed in any one of claims 1 to 4, **characterized in that** said tag is selected from the group constituted by a luminescent agent, a radioisotope, an enzyme, biotin, acrylamide, a thiol and a phosphorothioate.

6. A method for detecting a genetic polymorphism of a single or of a plurality of base pair(s), comprising the following steps:
i) bringing a target nucleic acid that might contain one or more polymorphic bases to be detected into contact with a circularizable single-stranded DNA (probe);
ii) hybridizing said single-stranded DNA using the target nucleic acid;
iii) if the target nucleic acid contains said polymorphic base or bases, obtaining a hybridized and circularized single-stranded DNA by ligation of said hybridized single-stranded DNA with the target nucleic acid in the presence of a DNA ligase;
iv) obtaining periodic double-stranded DNAs by hyperbranched rolling circle amplification of said hybridized and circularized single-stranded DNA in the presence of at least one of two primers with sequences 5'-(F1)ₙ₁-T1-E1-A1-3' and 5'-(F2)ₙ₂-T2-E2-A2-3' as defined in claims 1 to 5;
v) detecting the barcode sequences T1 and/or T2 present at the ends of the double-stranded periodic nucleic acids obtained in step iv) by hybridization with a nucleotide probe complementary to said barcode sequences T1 and/or T2 , wherein said nucleotide probe is present on a solid support ; detection of the barcode sequences T1 and/or T2 present at the ends of said periodic double-stranded DNAs indicating that said target nucleic acid contains said polymorphic base or bases.

7. The method as claimed in claim 6 , **characterized in that** the steps i) to iv) are carried out by mixing said circularizable single-stranded DNA, said target nucleic acid, said primers, deoxynucleotides, a DNA ligase, rATPs, a DNA polymerase deprived of exonuclease activity and having a strand displacement activity, a stabilizer for said polymerase, and an appropriate buffer in which said DNA ligase circularizes the single-stranded DNA hybridized to the target DNA if hybridization contains no mismatches, and said DNA polymerase catalyzes the polymerization of the DNA strands.

8. The method as claimed in claim 7, **characterized in that** said buffer comprises or is constituted by potassium acetate, tris-acetate, magnesium acetate and dithiothreitol.

9. The method as claimed in any one of claims 1 to 8, **characterized in that** said barcode sequences T1 and/or T2 are detected by a hybridization detection technique in which the detection signal for hybridization between the barcode sequence and a nucleotide sequence (probe) complementary to said barcode sequence increases with the number of nucleotides of the nucleic acid molecule comprising said barcode sequence.

10. The method as claimed in claim 9, **characterized in that** said barcode sequences T1 and/or T2 are detected by an electronic detection method.

11. Use of at least one pair of forward and reverse primers for the detection of a circularized single-stranded DNA by hyperbranched rolling circle amplification (HRCA);
wherein the forward primer is capable of hybridizing with said circularized single-stranded DNA and with negative strands generated during the HRCA, and the reverse primer is capable of hybridizing with positive strands generated during the HRCA;
and wherein said forward primer is constituted by or comprises the following sequence, from its 5' end to its 3' end: 5'-(F1)ₙ₁-T1-E1-A1-3', as defined in any one of claims 1 to 5 and/or said reverse primer is constituted by or comprises the following sequence, from its 5' end to its 3' end: 5'-(F2)ₙ₂-T2-E2-A2-3', as defined in any one of claims 1 to 5;
and a solid support comprising nucleotide sequences that are complementary to the barcode sequence.

12. A kit or set for detecting a genetic polymorphism of one or more base pair(s), **characterized in that** it comprises at least one pair of forward and reverse primers in which said forward primer is constituted by or comprises the following sequence, from its 5' end to its 3' end: 5' (F1)ₙ₁-T1-E1-A1-3', as defined in any one of Claims 1 to 5 and/or said reverse primer is constituted by or comprises the following sequence, from its 5' end to its 3' end: 5'-(F2)ₙ₂-T2-E2-A2-3', as defined in any one of claims 1 to 5, a circularizable single-stranded DNA, a DNA ligase, a DNA polymerase deprived of exonuclease activity and having a strand displacement activity, deoxynucleotides, an appropriate buffer and a microarray; wherein said microarray supports nucleotide sequences that are complementary to the barcode sequence.
